# EUROPEAN PATENT SPECIFICATION

(11) **EP 2 324 108 B1**
(45) Date of publication and mention of the grant of the patent: **09.07.2014**
(21) Application number: 09806437.1
(22) Date of filing: 13.08.2009
(51) Int. Cl.: C12N 1/20, C12R 1/40, A01N 63/00

(54) **MICROORGANISM CAPABLE OF SOLUBILIZING PHOSPHATE AND IRON AND ITS APPLICATIONS**
MIKROORGANISMUS MIT FÄHIGKEIT ZUM LÖSEN VON PHOSPHAT UND EISEN UND DESSEN ANWENDUNGEN
MICRO-ORGANISME SUSCEPTIBLE DE SOLUBILISER LE PHOSPHATE ET LE FER ET APPLICATIONS CORRESPONDANTES

(30) Priority: 14.08.2008 EP 08380251
(43) Date of publication of application: 25.05.2011
(73) Proprietor: Bio-Iliberis Research and Development S.L., 18100 Armilla, Granada (ES)
(72) Inventor: PIZARRO TOBÍAS, Paloma, 18100 Armilla (Granada) (ES); RAMOS DUQUE, Estrella, 18100 Armilla (Granada) (ES)
(74) Representative: ABG Patentes, S.L.
(86) International application number: PCT/EP2009/060503
(87) International publication number: WO 2010/018210

(56) References cited:
- DD-A5- 295 152
- US-A- 4 589 226
- ROSAS S B ET AL: "Phosphate-solubilizing Pseudomonas putida can influence the rhizobia-legume symbiosis" SOIL BIOLOGY AND BIOCHEMISTRY, vol. 38, no. 12, 1 December 2006 (2006-12-01), pages 3502-3505, XP005713289 ISSN: 0038-0717 [retrieved on 2006-12-01]
- CHABOT ROCK ET AL: "Growth stimulation of corn and romaine lettuce by inorganic phosphorus-solubilizing microorganisms" CANADIAN JOURNAL OF MICROBIOLOGY, vol. 39, no. 10, 1993, pages 941-947, XP002511357 ISSN: 0008-4166
- DATABASE WPI Week 200401 Thomson Scientific, London, GB; AN 2004-003083 XP002511360 & JP 2003 304862 A (KAGAKU GIJUTSU SHINKO JIGYODAN) 28 October 2003 (2003-10-28)
- SHEKHAR NAUTIYAL C: "An efficient microbiological growth medium for screening phosphate solubilizing microorganisms" FEMS MICROBIOLOGY LETTERS, vol. 170, no. 1, 1 January 1999 (1999-01-01), pages 265-270, XP002511358 ISSN: 0378-1097 cited in the application
- NAHAS E: "Factors determining rock phosphate solubilization by microorganisms isolated from soil" WORLD JOURNAL OF MICROBIOLOGY AND BIOTECHNOLOGY, vol. 12, no. 6, 1996, pages 567-572, XP002511359 ISSN: 0959-3993 cited in the application
- HAMDALI ET AL: "Screening for rock phosphate solubilizing Actinomycetes from Moroccan phosphate mines" APPLIED SOIL ECOLOGY, vol. 38, no. 1, 1 November 2007 (2007-11-01), pages 12-19, XP022324363 ISSN: 0929-1393
- ZHUANG ET AL: "New advances in plant growth-promoting rhizobacteria for bioremediation" ENVIRONMENT INTERNATIONAL, vol. 33, no. 3, 28 February 2007 (2007-02-28), pages 406-413, XP005909008 ISSN: 0160-4120
- GLICK B R: "Phytoremediation: synergistic use of plants and bacteria to clean up the environment" BIOTECHNOLOGY ADVANCES, vol. 21, no. 5, 1 August 2003 (2003-08-01), pages 383-393, XP004438924 ISSN: 0734-9750
- ZAHIR Z A ET AL: "Effectiveness of rhizobacteria containing ACC deaminase for growth promotion of peas (Pisum sativum) under drought conditions" JOURNAL OF MICROBIOLOGY AND BIOTECHNOLOGY, vol. 18, no. 5, May 2008 (2008-05), pages 958-963 URL, XP002554970 ISSN: 1017-7825(print) 1738-8872(ele

### SEQUENCE LISTING

<110> Bio-ILIBERIS Research and Development S.L.
<120> Microorganism capable of solubilizing phosphate and iron and its applications
<130> P3795PC00
<150> EP08380251.2
   <151> 2008-08-14
<160> 3
<170> PatentIn version 3.5
<210> 1
   <211> 20
   <212> DNA
   <213> Artificial
<220>
   <223> Primer F8
<400> 1
   agagtttgat cctggctcag 20
<210> 2
   <211> 15
   <212> DNA
   <213> Artificial
<220>
   <223> Primer R798
<400> 2
   ggggtatcta atccc 15
<210> 3
   <211> 105
   <212> DNA
   <213> Pseudomonas putida strain BIRD-1 (CECT 4715)
<400> 3
   gcaagtcgag cggatgacgg gagcttgctc cttgattcag cggcggacgg gtgagtaatg   60
   cctaggaatc tgcctggtag tgggggacaa cgtttcgaaa ggaac                  105

## Description

### Field of the Invention

The invention relates to a novel *Pseudomonas putida* strain capable of solubilizing phosphate and iron, and optionally producing plant growth-promoting compounds, or a mutant of said microorganism having essentially the same characteristics as the parent strain, and to its applications, particularly in solubilizing insoluble phosphate, in chelating iron to prevent pathogen growth and optionally in producing plant hormones. The novel *P. putida* strain and mutants thereof are capable of solubilizing phosphate and iron, and optionally producing plant growth-promoting compounds, in conditions wherein the amount of water is 20% or lower.

### Background of the Invention

The use of fertilizers has been and is a determining factor in the yield of plant crops of agricultural interest, the use of inorganic fertilizers containing mixtures of phosphorus, potassium and nitrogen (known as NPK) being frequent. The production of phosphates by the chemical industry is based on etching rock phosphate with a strong acid, for example sulfuric acid, giving rise to the formation of gypsum-type insoluble residues. An additional problem of inorganic fertilizers is that the phosphorus frequently precipitates (particularly in slightly alkaline soils) and gives rise to the insolubilization of the same. NPK-type fertilizers can also be obtained by means of the mixture of rock phosphate with ammonium sulfate and potassium chloride. In all these cases it results in a form of phosphorus in the soil which is not found in a readily bioavailable state for plants. In addition, these fertilizers do not take into account other possible nutrient deficiencies in the soil as is the case of iron which, being a quantitatively abundant element in the earth's crust due to its degree of oxidation, is not found in a bioavailable state for plants either.

In contrast with the chemical approaches of solubilizing the phosphorus in the rock phosphate, the biological approaches are based on the production of weak acids by microorganisms. Solutions to said problem using microorganisms which solubilize phosphorus *in situ* and making it bioavailable for plants have been proposed; however such approaches are partial and do not consider the global dimension or the solubilization of more than one nutrient which facilitates the growth of plants.

Microbiology in general and Biotechnology in particular provide a biological alternative to the solubilization of phosphorus by means of the use, by suitable microorganisms, of the phosphate present in rock phosphate as a phosphorus source. Several studies include the possibility of solubilizing phosphate of soils by microorganisms. Among these studies, those which achieve solubilizing phosphorus due to the excretion of acids which facilitate the solubilization of the insoluble phosphate, a phenomenon associated to fungi, e.g., *Penicillium* and *Aspergillus,* deserve special mention (Pea, A, Kucey, RMN and Stewart, JWB; Inorganic phosphate solubilization by two Penicillium species in solution culture and soil. Soil Biology and Biochemistry (1981), 20:459-464; E. Nahal. Factors determining rock phosphate solubilization by microorganisms isolated from soil. World J. Microbiol. Biotechnol. (1996), 12:567-572; and Vassileu N., Baca, M.T., Yassileva M., Franco I., and Azcón, R. Rock phosphate solubilization by Aspergillus niger grown on sugar-beet waste medium. Appl. Microbiol. Biotechnol. (1995), 44:546-549). Bacteria have also been described which form symbiosis with the root system of plants capable of solubilizing phosphates, e.g. *Rhizobium* and *Bradyrhizobium* genera strains (Halder, A.K., Mishra, A.K., Battacharyya, P., and Chakrabarty, P.K. Solubilization of rock phosphate by Rhizobium and Bradyrhizobium. J. Gene. Microbiol. (1990), 36:81-92), potentially pathogenic bacteria such as *Pseudomonas cepacia* (E. Nahal mentioned above), as well as bacteria not assigned to any genus, such as those identified as NBRI2601, NBRI3246 and NBR4003 by Nautilaya et al. (Nautilaya, C. S., B. Bhadauria, P. Kumar, H. Lal, R. Mondel and D. Verma. Stress induced phosphate solubilization in bacteria isolated from alkaline soils. FEMS Microbiol. Lett. (2000), 182:291-296) or some *Pseudomonas* sp. strains (Illmer, P. and Schinner, F. Solubilization of inorganic calcium phosphates - solubilization mechanisms. Soil Biology and Biochemistry (1995), 27:257-263).

It is also desirable to point out that most of iron in the earth's crust is found in insoluble forms that are difficult for microorganisms to access; in fact it has been described that iron can act as a limiting factor for the growth of microorganisms in their specific habitats. Some microorganisms are capable of producing molecules called siderophores having a high affinity for iron, such as *Pseudomonas aeruginosa* (Cox, C.D., Adams, P. Siderophore activity of pyoverdin for Pseudomonas aeruginosa. Infection and Immunity. (1985), 48:130-138) and *Pseudomonas fluorescens* (Duffy, B. K., Défago, G. Environmental factors modulating antibiotic and siderophore biosynthesis by Pseudomonas fluorescens biocontrol strains. Applied and Environmental Microbiology. (1999), 65:2429-2438). These molecules are excreted into the environment and, in the presence of iron, the siderophore-iron complex is formed. This complex is incorporated by the microorganism into the cell, thus preventing bioavailability of the iron for competing microorganisms. This property is particularly important since it aids in suppressing the pathogen growth. In fact, iron is an essential factor so that the virulence of certain strains is manifested both in models of animal and plant systems. Among those works which have studied this issue, one work relating to the study of the regulation of the synthesis of these molecules deserves special mention (Ventura, V., Weisbeek, P. and Koster, M. Gene regulation of siderophore-mediated iron acquisition in Pseudomonas: not only the Fur repressor. Molecular Microbiology. (1995), 17:603-610), as does another work relating to the diversity of siderophores (Cornels, P. and Matthijs, S. Diversity of siderophore-mediated iron uptake systems in fluorescent Pseudomonas: not only pyoverdins. Environ. Microbiol. (2002), 4:787-798).

Rosas S.B. et al. (Rosas S.B. et al. Phosphate-solubilizing Pseudomonas putida can influence the rhizobia-legume symbiosis. Soil Biology & Biochemistry (2006), 38:3502-3505) disclose two strains of P. *putida* capable of solubilizing phosphate and iron in non-low humidity conditions (i.e., inoculums of said strains were applied to the root system of some seedlings in the planting hole and the plants were suitable watered and grown under controlled conditions).

Further, for the purpose of increasing the value of microorganisms which solubilize phosphates and iron it is advantageous to identify those which furthermore produce compounds having a beneficial effect on the plant growth (Bloemberg, G.V., and Lugtenberg, B.J.J. Molecular basis of plant growth promotion and biocontrol by rhizobacteria. Curr. Opin. in Plant Biol. (2001), 4:434-450).

Additionally, the value of said microorganisms which solubilize phosphates and iron, and, optionally produce compounds having a beneficial effect on the plant growth, would increase if said microorganisms were tolerant towards dessication, i.e., if they were able to survive under low, preferably, under extreme low humidity conditions, e.g., in soils with only 2% field capacity, because said microorganisms would be able to survive during periods in which water is limited and could be used in regions wherein pluviometry is scarce.

It is therefore of interest to have microorganisms capable of solubilizing phosphate and iron under extreme low humidity conditions; advantageously, said microorganisms could furthermore produce compounds with plant growth-promoting properties under such humidity conditions.

### Summary of the Invention

*A Pseudomonas putida* strain *(P. putida* BIRD-1) has been isolated and characterized and deposited on June 3, 2008 in the Spanish Type Culture Collection (CECT) with access number CECT 7415. Said strain is capable of solubilizing phosphate and iron present in rock phosphate and of using said compounds as a phosphorus and iron source, respectively, whereby it can be used in a microbiological process for the solubilization of phosphate and iron. Surprisingly, said strain is capable of solubilizing phosphate and iron in extreme low humidity conditions, e.g., in soils with only 2% field capacity. It has further been observed that said strain also produces compounds with potential as plant hormones since they are capable of stimulating plant growth.

The invention therefore also contributes not only to preventing the environmental problems associated to the use of fertilizers based on rock phosphate by means of the use of microorganisms which solubilize insoluble phosphate and insoluble iron, overcoming the drawbacks of the processes of the state of the art, but rather it can also contribute to preventing the development of pathogenic organisms, plant pathogens in particular, and to stimulating plant growth. In addition, the invention further provides microorganisms which are extremely tolerant to extreme low humidity conditions which can survive in extreme low humidity conditions and can be used in regions wherein pluviometry is scarce.

Therefore, in an aspect, the invention relates to a microorganism of the *Pseudomonas putida* species deposited in the CECT with access number CECT 7415, having phosphate and iron solubilizing capacity in conditions wherein the amount of water is 20% or lower, or a mutant of said microorganism maintaining phosphate and iron solubilizing capacity in conditions wherein the amount of water is 20% or lower. A biologically pure culture of said microorganism is an additional aspect of this invention.

In another aspect, the invention relates to an active solid support comprising a solid support and said microorganism.

In another aspect, the invention relates to a supplemented solid fertilizer comprising a solid fertilizer and said microorganism.

In another aspect, the invention relates to a supplemented seed comprising a seed and said microorganism.

In another aspect, the invention relates to a process for the microbiological solubilization, under aerobic conditions, of a phosphate in a solid substrate containing an insoluble phosphate.

In another aspect, the invention relates to a process for the microbiological solubilization, under aerobic conditions, of insoluble phosphate present in a soil.

In another aspect, the invention relates to a process for the microbiological solubilization, under aerobic conditions, of insoluble iron present in a solid substrate containing insoluble iron.

In another aspect, the invention relates to a process for the microbiological solubilization, under aerobic conditions, of insoluble iron present in a soil containing iron oxides and other insoluble forms of this metal.

In another aspect, the invention relates to a process for the microbiological solubilization, under aerobic conditions, of an insoluble phosphate and of insoluble iron in a solid substrate containing an insoluble phosphate and insoluble iron.

In another aspect, the invention relates to a process for the microbiological solubilization, under aerobic conditions, of an insoluble phosphate and of insoluble iron present in a soil containing an insoluble phosphate and insoluble iron.

In another aspect, the invention relates to a process for promoting plant growth based on the use of the above mentioned microorganism.

In a particular embodiment, the above mentioned processes are carried out in conditions wherein the amount of water is 20% or lower.

In another aspect, the invention relates to a process for isolating a microorganism having phosphate and iron solubilizing capacity in extreme low humidity conditions.

### Detailed Description of the Invention

The invention generally relates to bacteria of the *Pseudomonas* genus with phosphate and iron solubilizing capacity in extreme low humidity conditions, and to its applications, particularly to its use in the microbiological solubilization of phosphate and iron, and, optionally, in the production of compounds with potential as plant hormones useful for stimulating plant growth. Said bacteria are particularly capable of both using insoluble phosphates as a phosphorus source as well as also solubilizing insoluble iron by means of, for example, producing siderophores with iron chelating properties which, after forming complexes with iron, are subsequently reassimilated by the bacteria to release iron inside said bacteria. The plants could alternatively incorporate the chelated forms of iron. Given that iron is an essential element in many physiological processes, its incorporation facilitates the growth of bacteria and furthermore limits the availability of iron for pathogens, which contributes to reducing the pathogen load.

Further, the microorganism provided by the instant invention is extremely tolerant to extreme low humidity conditions due to the presence of a number of specific genes that exhibit tolerance towards desiccation, and solubilizes phosphate and produces siderophores in such conditions. Moreover, due to the formation of superficial structures, the microorganism is able to adhere to seeds under any humidity condition.

### 1. Microorganism of the invention

In an aspect, the invention relates to a microorganism, hereinafter microorganism of the invention, of the *Pseudomonas* putida species deposited in the Spanish Type Culture Collection (CECT) with access number CECT 7415, having phosphate and iron solubilizing capacity in extreme low humidity conditions, or a mutant of said microorganism maintaining phosphate and iron solubilizing capacity in conditions wherein the amount of water is 20% or lower.

In a particular embodiment, the microorganism of the invention furthermore produces plant growth-stimulating compounds in conditions wherein the amount of water is 20% or lower.

These capacities jointly [solubilization of phosphate and iron (and, optionally, production of plant growth-stimulating compounds) under extreme low humidity conditions] are not associated to the *P. putida* species.

The amount of phosphate which solubilizes said microorganism *P*. *putida* CECT 7415 can reach up to 10% of the total present in rock phosphate in 24 hours. Even though said microorganism *P*. *putida* CECT 7415 promotes plant growth in excess phosphate conditions, the nature of the hormone or precursor is unknown.

As used herein, the expression "phosphate and iron solubilizing capacity" means that the microorganism of the invention is capable of (i) solubilizing insoluble phosphate, such as the phosphate present, for example, in soils or in rock phosphate [e.g., dibasic calcium phosphate (CaHPO₄.2H₂O), tribasic calcium phosphate (Ca₅(OH)(PO₄)₃, etc.], and using it as a phosphorus source, and also of (ii) solubilizing insoluble iron present, for example, in soils or in rock phosphate [e.g., iron oxides, etc.]; the most frequent way that the microorganisms solubilize iron is by means of the production of siderophores with iron chelating properties; said siderophores, in the presence of iron, form siderophore-iron complexes which are reassimilated by the microorganism of the invention to release the iron inside the same.

A microorganism's phosphate solubilizing capacity can be determined by any conventional process, for example, inoculating a culture of said microorganism in a medium containing one or more insoluble phosphates as the sole phosphorus source (e.g., tricalcium phosphate, etc.), incubating under suitable conditions and counting the viable microorganisms, as described in Examples 1-3 enclosed with the present description; under these conditions, the maintenance or an increase of the bacterial population density is indicative that said microorganisms solubilize said insoluble phosphate/phosphates and use it/them as a phosphorus source (Rodriguez, H., Fraga, R. Phosphate solubilizing bacteria and their role in plant growth promotion. Biotechnology Advances. (1999), 17:319-339). Another assay which allows clarifying if a microorganism is capable of solubilizing insoluble phosphates comprises seeding them in solid media specialized for this purpose, in which the microorganism generates a microenvironment due to the production of organic acids, which translates into a visible halo in the medium (Nautiyal, C.S. An efficient microbiological growth medium for screening phosphate solubilizing microorganisms. Microbiology Letters. (1999), 170 (1): 265-270).

A microorganism's iron solubilizing capacity can be determined by any conventional process, for example, inoculating a culture of said microorganism in a suitable culture medium without adding iron, incubating under suitable conditions and counting the viable microorganisms, as described in Examples 1 and 4 enclosed with the present description; under these conditions, the maintenance or an increase of the bacterial population density is indicative that said microorganisms use iron, or, the formation of a greenish-yellow color in the supernatants of the cultures is indicative of the excretion of certain siderophores into the culture medium. The processes of solubilizing phosphates and iron are of an independent genetic nature since mutants can be obtained which solubilize phosphate and not iron and vice versa.

As used herein, the expression "extreme low humidity conditions" means that the microorganism of the invention is tolerant to conditions in which the amount of water is 20% or lower, typically 10% or lower, usually 5% or lower, e.g., about 2%. Said expression "extreme low humidity conditions" applied to soils means a low field capacity, such as soils with 10% field capacity or less, for example, soils with 5% field capacity or less, e.g., soils with 2% field capacity. As it is known, "field capacity" is the amount of soil moisture or water content held in soil after excess water has drained away and the rate of downward movement has materially decreased, which usually takes place within 2-3 days after a rain or irrigation in previous soils of uniform structure and texture. Field capacity is a constant characteristic for each soil and depends mainly on the texture, amount of organic matter and compactation degree thereof.

The microorganism of the invention remains viable in extreme low humidity conditions allowing the solubilization of phosphate and iron in extreme low humidity conditions (Example 6). As a consequence thereof, the microorganism of the invention can be used to solubilise phosphate and/or iron, and, optionally, to produce plant growth-stimulating compounds, in extreme low humidity conditions, what constitutes an important advantage over microorganisms which are less tolerant towards dessication.

Of course, the microorganism of the invention also remains viable under other humidity conditions, for example, under high, normal or low humidity conditions, e.g., in soils with 50% field capacity or less, such as soils with 20% field capacity or less (Example 6) and even in soils with more than 50% field capacity. Therefore, the microorganism of the invention, which has a high tolerance towards dessication, can be used to solubilise phosphate and/or iron, and, optionally, to produce plant growth-stimulating compounds, not only in extreme low humidity conditions, but also in high, normal or low humidity conditions.

Tolerance towards dessication is an important quality in this kind of microorganisms since it involves that the microorganism (as the microorganism of the invention) has a higher ability to survive under conditions in which water is scarce or limited, e.g., under hydric stress conditions, since the microorganism is capable of remaining in soil during drought, dry seasons (e.g., summer), wherein non-tolerant towards dessication microorganisms cannot survive. Therefore, the application, handling and manipulation of the microorganism of the invention are simpler and cheaper; in addition, the microorganism of the invention can be used in regions wherein pluviometry is scarce.

In a particular embodiment, the microorganism of the invention is the P. *putida* BIRD-1 strain (CECT 7415).

In another particular embodiment, the microorganism of the invention is a mutant of said *P. putida* BIRD-1 strain (CECT 7415) maintaining phosphate and iron solubilizing capacity in conditions wherein the amount of water is 20% or lower. As used herein, the term "mutant" includes any individual or organism resulting from a mutation or change in the DNA of a gene of an organism resulting in a character (phenotype) that is not found in wild-type; in a specific embodiment, said mutant is a mutant of P. *putida* BIRD-1 (CECT 7415) maintaining essentially the same characteristics as those of the parent strain *[P. putida* BIRD-1 (CECT 7415)], and it further has phosphate and iron solubilizing capacity in extreme low humidity conditions.

The microorganism of the invention can be used for solubilizing, by means of a microbiological process, phosphate and iron, at practically any level of humidity (i.e., including in extreme low humidity conditions); in particular, said microorganism of the invention is capable of solubilizing both insoluble phosphate/phosphates, using it/them as a phosphorus source, and insoluble iron, by means of the production of siderophores with chelating properties which are subsequently reassimilated by the microorganism to release the iron inside the microorganism; iron is used by the microorganism as an essential element in many physiological processes and to facilitate growth of the microorganism, whereby said microorganism limits the availability of iron for pathogen growth and as a result pathogen growth is prevented or minimized. Both insoluble phosphate and insoluble iron can be found in any solid substrate containing an insoluble phosphate and/or insoluble iron, for example, soil, rock phosphate, solid fertilizers, etc.; any of said substrates can be used by the microorganism of the invention as a phosphorus and/or iron source. In a particular embodiment, the substrate contains insoluble phosphate but it does not contain iron. In another particular embodiment, the substrate contains insoluble iron but it does not contain insoluble phosphate. In another particular embodiment, the substrate contains both insoluble phosphate and iron.

The microorganism of the invention has been isolated from a garden soil sample. Briefly, a sample of said soil was suspended in a medium containing insoluble phosphate as the sole phosphorus source and the resulting suspension was incubated at a temperature comprised between 25°C and 30°C, with stirring for a week under aerobic conditions; after this time period, suitable dilutions of said suspension were seeded in plates of selective solid medium containing a carbon source, a nitrogen source and nutrients, which were incubated between 1 and 7 days. The isolated colonies were purified and classified as *P. putida* (see below). Said bacteria were subsequently inoculated in an iron-deficient medium, incubated with stirring and the colonies of the cultures the supernatants of which acquired a greenish-yellow color typical of the excretion of siderophores into the culture medium, as indicated in Example 1, were selected. One of the isolated colonies, the most efficient one, was the so-called *P. putida* BIRD-1, which has been deposited in the CECT, with access number CECT 7415.

Following a microbiology-type analysis, the most efficient bacterium has been assigned to the *Pseudomonas* genus. The isolated bacterium is rod-shaped and shows no pigmentation when cultured in plates of minimal medium or LB enriched medium (Maniatis et al. Molecular Cloning, A Laboratory Manual, Cold Spring Harbor Laboratory, NY, 1982). Fructose, glucose, acetic acid, succinic acid, glycerol or benzoate can be used as a carbon source and ammonium compounds, nitrates and nitrites can be used as a nitrogen source in M9-type minimal medium.

It was confirmed that said BIRD-1 strain belongs to the P. *putida* species by means of analyzing the partial sequence of the gene encoding rRNA 16S, used as a phylogenetic marker, by means of using forward primer F8 (SEQ ID NO: 1) and reverse primer R798 (SEQ ID NO: 2), described by Liu et al. (Liu, Z., Lozupone, C., Hamady, M., Bushman, F.D. and Knight, R. Short pyrosequencing reads suffice for accurate microbial community analysis. Nucleic Acids Research. (2007), 35:e120), which amplify a specific DNA fragment of *P*: *putida* of 105 nucleotides (SEQ ID NO: 3). To that end, briefly, the total DNA was isolated from the strain *(P. putida* BIRD-1) by means of alkaline lysis, primers F8 (SEQ ID NO: 1) and R798 (SEQ ID NO: 2) were added, and in the presence of a Taq polymerase, a DNA fragment was amplified and subsequently sequenced. The determined sequence shows 100% of identity to that of different *P*. *putida* strains.

In an analysis of the genes of the *P*. *putida* BIRD-1 strain (CECT 7415), the *rpoD, gyrB, trpE, gltA* and *edd* genes have been either partially of totally amplified. The sequences of the genes show high similarity with respect to those of other strains of the species, although there are significant changes that indicate that it is a natural variant with specific genetic differences (Example 5).

In a particular embodiment, the microorganism of the invention furthermore has phosphate and iron solubilizing capacity, the capacity of producing plant growth-promoting compounds, such as, for example, compounds which facilitate the development of the root system, the elongation of stems, flowering, etc., at practically any level of humidity (even including extreme low humidity conditions).

The capacity of the microorganism of the invention of producing plant growth-stimulating compounds can be determined by any conventional process, for example, by means of an early plant growth stimulation assay. Briefly, said assay, which can be performed in a greenhouse, comprises sowing seeds (e.g., corn, barley, grass, AvexIII (mixture of pasture plants), etc.) in a homogenized mixture made up of agricultural soil mixed with sand (silica) and *P. putida* BIRD-1 bacteria (CECT 7415), preferably adhered on a solid support, and determining the percentage of seed germination and the stem length a week after sowing, as described in Examples 10 and 11. The capacity of P. *putida* BIRD-1 (CECT 7415) of producing plant growth-stimulating compounds was assayed by means of a seed germination stimulation assay comprising, briefly, sowing seeds (e.g., barley, corn, grass, AvexIII, etc.) in Petri dishes containing a suitable soil previously mixed with *P. putida* BIRD-1 bacteria (CECT 7415) in a suitable bacterial population density followed by incubation under conditions which allow the seed germination (e.g., 20°C in the dark), and determining the percentage of seed germination, as indicated in Examples 7-9.

A biologically pure culture of a microorganism of the invention is an additional aspect of the invention.

### 2. Isolation of microorganisms with phosphate and iron solubilizing capacity in extreme low humidity conditions

In another aspect, the invention relates to a process for isolating a microorganism, hereinafter isolation process of the invention, having phosphate and iron solubilizing capacity in conditions wherein the amount of water is 20% or lower, comprising:
a) suspending a sample of a soil suspected of containing said microorganism having phosphate and iron solubilizing capacity in a culture medium lacking a phosphorus source;
b) adding to the suspension obtained in step a) a phosphorus source formed by one or more insoluble phosphates, a carbon source and a nitrogen source;
c) incubating the suspension resulting from step b) under aerobic conditions which allow the growth of said microorganisms which solubilize the insoluble phosphate;
d) seeding dilutions of the suspension resulting from step c) in plates of selective solid medium containing up to 1% (w/w) of an insoluble phosphate as the sole phosphorus source;
e) incubating the seeded plates of step d) during a time period comprised between 1 and 7 days at a temperature comprised between 15°C and 42°C;
f) isolating and purifying the colonies of the microorganisms which, obtained after the incubation of step e), are capable of using insoluble phosphate in aerobic conditions;
g) optionally selecting and, if desired, characterizing the strains of the colonies of microorganisms isolated and purified in step f);
h) seeding in plates colonies of the microorganisms which, isolated and purified in step f) or optionally selected in step g), are capable of using insoluble phosphate under aerobic conditions, in an iron-deficient culture medium containing a carbon source and a nitrogen source;
i) incubating the seeded plates of step h) at a temperature comprised between 15°C and 42°C during a time period comprised between 1 and 7 days, under conditions allowing the growth of microorganisms which solubilize iron;
j) seeding dilutions of the suspension resulting from step i) on plates of iron-deficient solid culture medium;
k) incubating the seeded plates of step j) during a time period comprised between 1 and 7 days at a temperature comprised between 15°C and 42°C;
l) isolating and purifying the colonies of microorganisms which, obtained after the incubation of step k), are capable of obtaining iron from the trace concentrations present in the culture media and under aerobic conditions;
m) selecting and optionally characterizing the strains of the colonies of the microorganisms isolated and purified in step l); and
n) subjecting the strains of the colonies of the microorganisms selected and optionally characterized in step m) to an assay to assess tolerance towards desiccation and selecting those strains having phosphate and iron solubilizing capacity in conditions wherein the amount of water is 20% or lower.

As can be seen, the isolation process of the invention first of all comprises identifying microorganisms capable of solubilizing phosphate, and secondly identifying microorganisms which in addition to solubilizing insoluble phosphate, are capable of solubilizing insoluble iron; nevertheless, the person skilled in the art will understand that it is also possible to reverse the order, i.e., firstly identifying microorganisms capable of solubilizing insoluble iron and secondly identifying microorganisms which in addition to solubilizing insoluble iron, are capable of solubilizing insoluble phosphate, making the appropriate adaptations to isolate microorganisms capable of solubilizing phosphate and iron.

The sample of soil which can be used in step a) of the isolation process of the invention can be any sample of a soil suspected of containing microorganisms with phosphate and iron solubilizing capacity, for example, an agricultural soil, a garden soil, etc., suspected of containing such microorganisms. Said sample of soil is suspended in a culture medium lacking a phosphorus source, such as modified A medium (Nautilaya, C.S. An efficient microbiological growth medium for screening phosphate solubilizing microorganisms. FEMS Microbiol. Lett. (1999), 170:265-270) the specific composition of which per liter is: NH₄Cl, 267 mg; MgSO₄.7H₂O, 410 mg; KCl, 300 mg; NaCl, 200 mg and 1 ml of an aqueous solution of iron citrate (6 g/l).

According to step b), (i) a phosphorus source formed by one or more insoluble phosphates which are not usable by the microorganisms unless they are solubilized; (ii) a carbon source and (iii) a nitrogen source are added to the suspension obtained in step a).

Although virtually any insoluble phosphate can be used, e.g., dibasic calcium phosphate (CaHPO₄.2H₂O), tribasic calcium phosphate (Ca₅(OH)(PO₄)₃, etc., and mixtures thereof; in a particular embodiment a mixture of insoluble calcium phosphate comprising dibasic calcium phosphate and tribasic calcium phosphate is added at a 1:1 ratio (w:w). The amount of phosphate or insoluble phosphates which can be added to the suspension obtained in step a) may vary within a wide range; nevertheless, in a particular embodiment the amount of insoluble phosphate which is added to said suspension is comprised between 0.1 and 90 g per liter, for example, 0.1, 0.5, 1.5, 10, 25, 50, 75 or 90 g/l. In a specific embodiment, between 0.1 and 90 g per liter of a mixture of insoluble calcium phosphate comprising dibasic calcium phosphate and tribasic calcium phosphate is added at a 1:1 ratio (w:w).

Any carbon source assimilable by the microorganisms to be identified can be used as a carbon source, for example, glucose, benzoate, acetate, etc. The amount of carbon source which can be added to the suspension obtained in step a) may vary within a wide range; nevertheless in a particular embodiment, carbon source is added in a suitable amount to reach a concentration comprised between 10 and 20 mM.

Any nitrogen source assimilable by the microorganisms to be identified can be used as a nitrogen source, for example, ammonium compounds, nitrate, urea, amino acids, etc. The amount of nitrogen source which can be added to the suspension obtained in step a) may vary within a wide range; nevertheless in a particular embodiment, nitrogen source is added in a suitable amount to reach a concentration comprised between 5 and 10 mM.

In step c), the suspension resulting from step b) is incubated at a temperature comprised between 15°C and 42°C, preferably between 25°C and 30°C, during a time period comprised between 1 and 7 days, with optional stirring, under aerobic conditions allowing the growth of microorganisms which solubilize the insoluble phosphate.

Subsequently (step d)], dilutions of the suspension resulting from step c) are spread on plates of selective solid medium containing up to 1% (w/w) of an insoluble phosphate as the sole phosphorus source. Although virtually any insoluble phosphate can be used, e.g., dibasic calcium phosphate, tribasic calcium phosphate, etc., and mixtures thereof, in a particular embodiment, said phosphorus source comprises tribasic calcium phosphate; in another particular embodiment, said phosphorus source comprises dibasic calcium phosphate and tribasic calcium phosphate. Then the spread plates of step d) are incubated at a temperature comprised between 15°C and 42°C, preferably between 25°C and 30°C, during a time period comprised between 1 and 7 days [step e)], with optional stirring.

Subsequently in step f), the colonies of microorganisms which, obtained after the incubation of step e), are capable of using insoluble phosphate (e.g., dibasic calcium phosphate, tribasic calcium phosphate, etc., and mixtures thereof) under aerobic conditions are isolated and purified, and then if desired, the strains, preferably the most efficient strains, of the colonies of microorganisms isolated and purified in step f) are selected [step g)] and optionally characterized. In practice, it is advisable to characterize the microorganisms for the purpose of selecting the carbon and nitrogen sources to be added to the subsequent culture media. Bacteria of the *Pseudomonas, Alcaligenes, Agrobacterium, Mycobacterium, Rhizobium* genera, etc., have been identified; nevertheless in a particular embodiment, the most efficient strain was a *Pseudomonas* sp. strain.

The colonies of the microorganisms which, isolated and purified in step f) or optionally selected in step g), are capable of using insoluble phosphate (e.g., dibasic calcium phosphate, tribasic calcium phosphate, etc., and mixtures thereof) under aerobic conditions, are seeded (step h)] in an iron-deficient culture medium containing a carbon source and a nitrogen source but to which medium iron is not added. Although virtually any iron-deficient culture medium can be used, in a particular embodiment King's medium is used, the composition of which per liter is as follows: 10 g of meat peptone, 10 g of casein peptone; 1.5 g of KH₂PO₄; 1.5 g of solidified MgSO₄ with agar (15 g/l).

Any carbon source assimilable by the microorganisms to be identified can be used as a carbon source, for example, benzoate, glycerol, citrate, glucose, etc. In a particular embodiment, the microorganism belongs to the *Pseudomonas* genus and the carbon source comprises sodium benzoate, glycerol, sodium citrate, glucose or another assimilable carbon source, preferably sodium benzoate. The amount of carbon source which can be present in said iron-deficient medium may vary within a wide range; nevertheless in a particular embodiment, said carbon source is present at a concentration comprised between 10 and 20 mM. In a specific embodiment, the carbon source used is selected between 10 mM sodium benzoate, 15 mM glycerol, 10 mM sodium citrate, 16 mM glucose or any other assimilable carbon source at the suitable concentration.

Any nitrogen source assimilable by the microorganisms to be identified can be used as a nitrogen source, for example, ammonium compounds, nitrite, urea, amino acids, etc. The amount of nitrogen source which can be present in said iron-deficient medium may vary within a wide range; nevertheless in a particular embodiment, the nitrogen source is present at a concentration comprised between 5 and 10 mM.

The bacteria are spread using the streaking technique and it is determined that the cultures are pure.

Then in step i), the plates resulting from step h) are incubated at a temperature comprised between 15°C and 42°C, preferably between 20°C and 30°C, during a time period comprised between 1 and 7 days, with optional stirring, under conditions allowing the growth of microorganisms which solubilize insoluble iron. When said microorganisms are present, the formation of individual colonies is observed and, in the areas of the highest density, the formation of a yellow-green color is observed indicative of the production of pyoverdin-type siderophores. Pyoverdins are iron chelating agents which usually produce *Pseudomonas* from the fluorescent group (Cox, C.D., Adams, P. Siderophore activity of pyoverdin for Pseudomonas aeruginosa. Infection and Immunity. (1985), 48:130-138) and are formed by a chromophore derivative of 2,3-diamino-6,7-dihydroxyquinoline, a peptide half bound to the chromophore and a side chain bound to the nitrogen atom of position C-3 of the chromophore. The composition of the peptide chains is typical of the strains. Pyoverdins chelate iron in a 1:1 ratio.

Subsequently in step j), dilutions of the suspension resulting from step i) are spread on plates of iron-deficient solid culture medium, such as selective solid minimal A medium which does not contain added iron (King, E.O., Ward, W.K. and Raney, D.E. Media for the demonstration of pyocyanin and fluorescein, J. Lab. Clin. Med. (1954), 44:301-307) or in King's medium, and the seeded plates in step j) are incubated [step k)] during a time period comprised between 1 and 7 days at a temperature comprised between 15°C and 42°C, preferably between 20°C and 30°C, with optional stirring.

Then in step l), the colonies of microorganisms which, obtained after the incubation of step k), are capable of obtaining iron from the trace concentrations present in the culture media under aerobic conditions are isolated and purified, and then if desired, the strains, preferably the most efficient strains, of the colonies of microorganisms isolated and purified in step 1) are selected [step m)] and optionally characterized. Although bacteria of the *Pseudomonas, Alcaligenes, Agrobacterium, Mycobacterium, Rhizobium* genera, etc., capable of solubilizing insoluble phosphate and insoluble iron have been identified, in a particular embodiment the most efficient strain was the strain identified as *P. putida* BIRD-1, which has been deposited in the CECT with access number CECT 7415. Said strain has the advantage that it further produces plant growth-promoting compounds (compounds with plant hormone properties).

Further, in step n) the strains of the colonies of the microorganisms selected and optionally characterized in step m) are subjected to an assay to assess tolerance towards desiccation. Any conventional assay to assess tolerance towards desiccation can be used. In a particular embodiment, tolerance towards desiccation of the strains selected in step m) is determined in step n) by adding the strains to test to soils whose humidity is prefixed at different values of field capacity (e.g., 50, 20, 10 and 2%) and checking the number of viable cells of the strain after a determined period of time (e.g., 15 days), as it is disclosed in Example 6. Step n) allows to identify those strains which are more tolerant towards dessication as well as the relative humidity levels wherein the different strains can survive what is important to guarantee the viability of the strain.

In a particular embodiment, the microorganism having phosphate and iron solubilizing capacity obtained according to the isolation process of the invention is the one identified as *P. putida* BIRD-1 (CECT 7415). Further, as it is shown in Example 6, *P putida* BIRD-1 (CECT 7415) tolerates extremely low humidity conditions.

In another particular embodiment, the microorganism having phosphate and iron solubilizing capacity obtained according to the isolation process of the invention is a mutant of said P. *putida* BIRD-1 (CECT 7415) bacterium having essentially the same characteristics as the parent strain and it further has phosphate and iron solubilizing capacity in conditions wherein the amount of water is 20% or lower.

In another particular embodiment, the microorganism isolated according to the isolation process of the invention is capable of solubilising both phosphate and iron in conditions wherein the amount of water is 20% or lower (i.e., extreme dessication conditions), remaining viable even when humidity is as low as 2%.

### 3. Applications

The microorganisms with insoluble phosphate and insoluble iron solubilizing capacities provided by this invention can be used to solubilize insoluble phosphates and iron present in solid substrates (e.g., soils, rocks, etc.). An advantage of this invention is that the solubilization of phosphates and iron by the microorganisms of the invention can occur at practically any level of humidity. As used herein the expression "any level of humidity" means that the level of humidity is not restricted to any specific range or value; thus the humidity may be high, medium or low, and includes also extreme low humidity conditions.

Additionally, given that the microorganisms of the invention have in addition to phosphate and iron solubilizing capacity, the capacity of producing plant growth-promoting compounds, they can be used to stimulate seed germination and/or plant growth, particularly plant growth in the earlier stages. Again, an advantage of this invention is that the production of said plant growth-promoting compounds by the microorganisms of the invention can occur at practically any level of humidity.

To facilitate the application and incorporation of the microorganisms of the invention and for the purpose of promoting their proliferation and interaction with the plants, it is advantageous to fix or adhere said microorganisms on a suitable solid support.

Therefore, in another aspect the invention relates to an active solid support, hereinafter, active solid support of the invention, comprising a solid support and a microorganism of the invention. Said solid support is an inert solid support for the microorganisms of the invention and the plants and it must advantageously have a high specific surface area so that it can have a high adsorption capacity for adsorbing the microorganisms of the invention (Busscher, H.J. and Weerkamp, A.H. Specific and non-specific interactions in bacterial adhesion to solid substrate. FEMS Microbiol. Lett. (1999), 46:465-173). Virtually any solid support meeting said conditions can be used in the present invention; illustrative and non-limiting examples of solid supports which can be used include clay, e.g., bentonite, sepiolite, etc.; a green waste, e.g., cork powder, cellulose, etc.; talc, etc., and mixtures thereof, since they adsorb at least 10⁵ colony forming units (cfu) per g of solid support, typically at least 10⁶, advantageously at least 10⁷, preferably at least 10⁸, more preferably at least 10⁹, even more preferably at least 10¹⁰ cfu/g of solid support.

Said active solid support of the invention can be obtained by conventional methods by mixing said solid support with a culture containing the microorganism of the invention; said mixture is carried out in the suitable ratio depending on the cfu to be contained in 1 g of solid support. In a particular embodiment, said culture comprises at least 10³ cfu, typically at least 10⁵, advantageously at least 10⁷, preferably at least 10⁹, cfu. Generally the mixture of the solid support with the culture containing the microorganism of the invention can be carried out based on a widely variable weight:volume (w:v) ratio, depending on, among other factors, the cfu present in the culture and the cfu per g of solid support which are to be obtained in the active solid support of the invention; nevertheless in a particular embodiment, said ratio [solid support]:[culture containing the microorganism of the invention] is comprised between 1:0.01 and 1:1 (w:v); in a specific embodiment, 1 ml of culture containing 10⁷ of the microorganism of the invention is mixed with 1 gram of solid support.

In another aspect, the invention relates to a supplemented fertilizer, hereinafter supplemented fertilizer of the invention, comprising a fertilizer and a microorganism of the invention. Virtually any solid or even liquid fertilizer can be used; illustrative non-limiting examples of said solid fertilizers include solid NPK-type fertilizers, etc.; any liquid fertilizer with a pH comprised between 2 and 10 can also be used; illustrative non-limiting examples of said liquid fertilizers include fertilizers based on fulvic acids, liquid fertilizers with a different NPK composition, such as 16:2:4, 20:5:0 mixtures, etc., or different types of fertilizers in drops with an NPK composition of 8:9:9, 16:4:4, etc. The microorganism of the invention can be added as such to the fertilizer or they can be alternatively and preferably adhered to a solid support, i.e., in the form of an active solid support of the invention as previously mentioned.

Said supplemented fertilizer of the invention can be obtained by conventional methods by mixing said fertilizer with a culture containing the microorganism of the invention; said mixture is carried out in the suitable ratio depending on the cfu which is to be contained in the fertilizer. In a particular embodiment, said culture comprises at least 10³ cfu, typically at least 10⁵, advantageously at least 10⁷, preferably at least 10⁹, cfu. Generally the mixture of the fertilizer with the culture containing the microorganism of the invention can be carried out based on a widely variable weight:volume (w:v) ratio, depending on, among other factors, the cfu present in the culture and the cfu per unit of measure (g or ml) of fertilizer which is to be obtained in the supplemented fertilizer of the invention; nevertheless in a particular embodiment, said ratio [fertilizer]:[culture containing the microorganism of the invention] is comprised between 1:0.01 and 1:1 (w:v); in a specific embodiment, between 0.01 and 1 ml of culture containing 10⁷ of the microorganism of the invention are mixed with 1 gram of solid fertilizer; in another specific embodiment, 1 ml of culture containing 10⁷ of the microorganism of the invention is mixed with 1 ml of liquid fertilizer with a pH comprised between 2 and 10.

Likewise, if desired, a suitable adhesive can be used to facilitate adhesion of the microorganisms of the invention to the fertilizer. Virtually any agriculturally acceptable adhesive can be used; illustrative non-limiting examples of said adhesives include acacia gum, alginate-based polymers (e.g., calcium alginate, etc.), etc. In this case, the amount of adhesive which can be used may vary within a wide range; nevertheless in a particular embodiment, said adhesive is present in the supplemented fertilizer of the invention, in particular, in a supplemented solid fertilizer with microorganisms of the invention, provided by this invention, in a proportion equal to or less than 1% by weight of the total weight. The presence of the microorganism of the invention in the solid fertilizer would contribute not only to reducing the environmental problem relating to the accumulation of insoluble phosphate on arable soils since it would enable its assimilation, but it would also contribute to reducing pathogen growth on the treated surface since it would remove iron, a necessary element for pathogens; from the surrounding area. Solid fertilizers thus supplied would play a dual role in the development of plants, namely (i) facilitating nutrients and stimulating growth and (ii) preventing pathogen growth (e.g., fungi, bacteria, etc.) for plants.

In another aspect, the invention relates to a supplemented seed, hereinafter supplemented seed of the invention, comprising a seed and a microorganism of the invention. Virtually any seed of any plant can be used; illustrative non-limiting examples of said seeds include seeds of plants of agricultural interest, including plants of interest in human or animal consumption, plants of a decorative interest, plants of an energy interest, etc. (oats, barley, corn, wheat, grass, sorghum, rose bushes, geraniums, daisies, etc.). The microorganism of the invention can completely or partially coat the seed.

The supplemented seed of the invention can be obtained by conventional methods, e.g., immersing the seed in a suspension (or culture) comprising the microorganism of the invention, or alternatively spraying said suspension on the seeds, etc. The microorganism of the invention can be added as such to the seed or it can be adhered to a solid support, i.e., in the form of an active solid support of the invention as previously mentioned. As previously mentioned, in a particular embodiment said suspension (or culture) comprises, at least 10³ cfu, typically at least 10⁵, advantageously at least 10⁷, preferably at least 10⁹, cfu. Suitable conditions are generally selected to obtain a supplemented seed of the invention with the desired amount of microorganism of the invention. In a particular embodiment, said [seed]:[culture containing the microorganism of the invention] ratio is comprised between 1:0.01 and 1:1 (w:v); in a specific embodiment, 1 ml of culture containing 10⁷ of the microorganism of the invention is mixed with 1 gram of seeds.

Likewise, if desired, a suitable adhesive can be used to facilitate adhesion of the microorganisms of the invention to the seeds. Virtually any agriculturally acceptable adhesive can be used, as previously mentioned; illustrative non-limiting examples of said adhesives include acacia gum, alginate-based polymers (e.g., calcium alginate, etc.), etc.; said adhesive can be added in the suitable amount, e.g., 0.1% acacia gum or a 0.1% calcium alginate suspension which can contain the microorganisms of the invention trapped in the gel.

In another aspect, the invention relates to a process for the microbiological solubilization, under aerobic conditions, of an insoluble phosphate in a solid substrate containing an insoluble phosphate, comprising contacting said solid substrate to be treated with a microorganism of the invention. In a particular embodiment, said microorganism of the invention forms part of an active solid support of the invention. Therefore, in a particular embodiment, said process comprises contacting said solid substrate containing an insoluble phosphate to be treated with a culture of a microorganism of the invention; or alternatively with an active solid support of the invention.

In a specific embodiment, said process comprises:
a) inoculating a mixture comprising said solid substrate containing an insoluble phosphate to be treated and water with a culture comprising a microorganism of the invention; and if desired,
b) removing said microorganisms.

Virtually any solid substrate containing an insoluble phosphate can be treated according to this process for completely or partially reducing the insoluble phosphate content; nevertheless in a particular embodiment, said solid substrate containing an insoluble phosphate to be treated is rock phosphate, such as the rock phosphate used to produce phosphate fertilizers with a variable insoluble phosphate content.

To perform this process, the solid substrate containing an insoluble phosphate to be treated is mixed with water and the resulting mixture is contacted with a culture of a microorganism of the invention, or with an active solid support of the invention, at a bacterial population density which may vary within a wide range. In a particular embodiment, at least 10³ microorganisms of the invention per ml of water are inoculated. After inoculation the microorganisms of the invention are left to act and after 1 to 24 hours, if desired, said microorganisms are removed by conventional methods, for example by means of decantation, precipitation with electrolytes, centrifugation, etc. If desired, the removed microorganisms of the invention can be reused in a new process for the microbiological solubilization, under aerobic conditions, of an insoluble phosphate and/or insoluble iron, in a solid substrate or in a soil containing an insoluble phosphate and/or insoluble iron.

This process can be carried out in a reactor, pond or the like in which the solid substrate containing an insoluble phosphate to be treated is introduced, duly equipped with means for feeding and draining water, solid substrate, inoculation of microorganisms of the invention and recovery of the same. If necessary, the mixture (solid substrate, water and microorganisms of the invention optionally forming part of an active solid support of the invention) is supplemented with a carbon source and/or with a nitrogen source and/or essential nutrients, for the purpose of facilitating survival of the microorganisms of the invention. By way of illustration, suitable amounts of micronutrient solution along with suitable amounts of magnesium, cobalt and molybdenum, typically in the micromolar order, can be added to optimize the process; nevertheless, in any case the choice and amount of nutrients and micronutrients to be added will depend on the composition of the solid substrate containing an insoluble phosphate (e.g., rock phosphate) to be treated and on the microbiological demand.

This process can be used to solubilize all or part of the insoluble phosphate present in the solid substrate containing an insoluble phosphate, for example rock phosphate used for producing phosphate fertilizers, for the purpose of reducing the insoluble phosphate content present in said phosphate fertilizers obtained from said rock phosphate.

In another aspect, the invention relates to a process for the microbiological solubilization, under aerobic conditions, of insoluble phosphate present in a soil containing an insoluble phosphate, wherein said soil has any level of humidity, the process comprising contacting said soil to be treated containing an insoluble phosphate and having any level of humidity with a microorganism of the invention. In a particular embodiment, said process comprises:
injecting, one or more times, a culture of a microorganism of the invention into said soil containing an insoluble phosphate and having any level of humidity; or alternatively
adding to said soil containing an insoluble phosphate and having any level of humidity an active solid support of the invention; or alternatively
sowing said soil containing an insoluble phosphate and having any level of humidity with a supplemented seed of the invention, or alternatively
adding to said containing an insoluble phosphate and having any level of humidity a supplemented fertilizer of the invention, or alternatively
sowing said soil containing an insoluble phosphate and having any level of humidity with a supplemented seed of the invention.

In a specific embodiment, said process for the microbiological solubilization, under aerobic conditions, of insoluble phosphate present in a soil containing an insoluble phosphate and having any level of humidity is carried out by means of injecting microorganisms of the invention into the soil to be treated, for example by means of a process comprising:
a) the primary injection into said soil of a culture containing a microorganism of the invention; and
b) successive injections of cultures containing microorganisms of the invention until the complete or partial solubilization of the insoluble phosphate present in the soil to be treated.

Virtually any soil containing an insoluble phosphate (e.g., dibasic calcium phosphate, tribasic calcium phosphate, etc., and mixtures thereof) can be treated according to this process for completely or partially reducing the insoluble phosphate content present in said soil to be treated; nevertheless in a particular embodiment, said soil is a agricultural topsoil, a topsoil treated with fertilizers, etc. If necessary, the soil to be treated is supplemented with a carbon source and/or with a nitrogen source and/or with essential nutrients to facilitate survival of the microorganisms of the invention.

The level of humidity of the soil can vary within a broad range (high, normal, low or extreme low humidity conditions); thus, soils with 50% field capacity or less, such as soils with 20% field capacity or less, e.g., soils with 10% field capacity or less, even soils with 2% field capacity can be used for carrying out the above defined process, as well as soils with more than 50% field capacity. This is due to the particulars of the microorganism of the invention which is extremely tolerant towards dessication and remains viable and functional at extreme low humidity conditions, as in soils with only 2% field capacity. Said particulars constitute an important advantage of the microorganism of the invention over other microorganisms which are less tolerant towards dessication.

The microorganisms of the invention are injected for the purpose of reaching high cell density in the soil to be treated, for example equal to or greater than 10³ microorganisms of the invention per gram of soil to be treated, typically equal to or greater than 10⁴ microorganisms of the invention per gram of soil to be treated, preferably equal to or greater than 10⁵ microorganisms of the invention per gram of soil to be treated, for the purpose of facilitating solubilization of the insoluble phosphate.

In a specific embodiment, said process for the microbiological solubilization, under aerobic conditions, of insoluble phosphate present in a soil containing an insoluble phosphate and having any level of humidity, is carried out by means of adding to said soil to be treated an active solid support of the invention comprising a microorganism of the invention. The features of said active solid support of the invention have been previously described. The amount of active solid support of the invention which is added to the soil to be treated (soil containing an insoluble phosphate and having any level of humidity) may vary within a wide range; nevertheless in a particular embodiment, the amount of said active solid support of the invention which is added to the soil to be treated is equal to or greater than 0.01 kg of active solid support of the invention per hectare (Ha) of soil to be treated, typically equal to or greater than 0.1 kg/Ha, preferably equal to or greater than 0.5 kg/Ha of soil to be treated, even more preferably equal to or greater than 1 kg/Ha of soil to be treated, for the purpose of facilitating solubilization of the insoluble phosphate.

In another specific embodiment, said process for the microbiological solubilization, under aerobic conditions, of insoluble phosphate present in a soil containing an insoluble phosphate and having any level of humidity, is carried out by means of adding to said soil to be treated a supplemented fertilizer of the invention comprising a microorganism of the invention. The features of said supplemented fertilizer of the invention have been previously described. The amount of supplemented fertilizer of the invention which is added to the soil to be treated (soil containing an insoluble phosphate and having any level of humidity) may vary within a wide range, depending on, among other factors, the amount of insoluble phosphate present in the soil to be treated and on the amount of microorganisms of the invention present in the supplemented fertilizer of the invention.

In another specific embodiment, said process for the microbiological solubilization, under aerobic conditions, of insoluble phosphate present in a soil containing insoluble phosphate and having any level of humidity is carried out by means of sowing said soil to be treated with a seed of the invention comprising a microorganism of the invention. The features of said seed of the invention have been previously described. The amount of supplemented seeds of the invention which are sowed in the soil to be treated (soil containing insoluble phosphate and having any level of humidity) may vary within a wide range, depending on the density required by the farmer.

The previously described processes for the microbiological solubilization of insoluble phosphates provide a number of advantages, including the following:
- high specificity in the solubilization of insoluble phosphates;
- they work in a wide range of phosphate concentrations, typically between 0.01% and 95% (w/w) phosphate; and
- they are highly versatile since they can be used *in situ* to solubilize said compounds in soils (having any level of humidity, even extreme low humidity conditions) or in reactors for solubilizing phosphate, which can be used as fertilizer per *se* or in mixtures with other compounds.

In another aspect, the invention relates to a process for the microbiological solubilization, under aerobic conditions, of insoluble iron in a solid substrate containing insoluble iron, comprising contacting said solid substrate with a microorganism of the invention. In a particular embodiment, said microorganism of the invention forms part of an active solid support of the invention. Therefore, in a particular embodiment said process comprises contacting said solid substrate containing insoluble iron to be treated with a culture of a microorganism of the invention; or alternatively with an active solid support of the invention.

In a specific embodiment, said process comprises:
a) inoculating a mixture comprising said solid substrate containing insoluble iron to be treated and water with a culture comprising a microorganism of the invention; and, if desired,
b) removing said microorganisms.

Virtually any solid substrate containing insoluble iron (e.g., insoluble iron of the iron oxide type or any other form of iron) can be treated according to this process for completely or partially reducing the insoluble iron content present in said solid substrate to be treated; nevertheless in a particular embodiment, said solid substrate containing insoluble iron to be treated is a soil, such as a agricultural topsoil, a topsoil treated with fertilizers, etc.

To carry out this process, the solid substrate containing insoluble iron to be treated is mixed with water and the resulting mixture is contacted with a culture of a microorganism of the invention, or with an active solid support of the invention, at a bacterial population density which may vary within a wide range. In a particular embodiment, at least 10³ microorganisms of the invention per ml of water are inoculated. After inoculation, the microorganisms of the invention are left to act and after 1 to 24 hours, if desired, said microorganisms are removed by conventional methods, for example, by means of decantation, precipitation with electrolytes, centrifugation, etc. If desired, the removed microorganisms of the invention can be reused in a new process for the microbiological solubilization, under aerobic conditions, of insoluble phosphate and/or insoluble iron in a solid substrate or in a soil containing insoluble phosphate and/or insoluble iron.

This process can be carried out in a reactor, pond or the like, in which the solid substrate containing insoluble iron to be treated is introduced, duly equipped with means for feeding and draining water, solid substrate, inoculation of microorganisms of the invention and recovery of the same. If necessary, the mixture (solid substrate, water and microorganisms of the invention optionally forming part of an active solid support of the invention) is supplemented with a carbon source and/or with a nitrogen source and/or essential nutrients, for the purpose of facilitating survival of the microorganisms of the invention. By way of illustration, suitable amounts of micronutrient solution along with suitable amounts of magnesium, cobalt and molybdenum, typically in the micromolar order, can be added to optimize the process; nevertheless, in any case the choice and amount of nutrients and micronutrients to be added will depend on the composition of the solid substrate containing insoluble iron to be treated and on the microbiological demand.

This process can be used to solubilize all or part of the insoluble iron present in the solid substrate containing insoluble iron, for the purpose of reducing the insoluble iron content present in said solid substrate.

In another aspect, the invention relates to a process for the microbiological solubilization, under aerobic conditions, of insoluble iron present in a soil containing insoluble iron, wherein said soil has any level of humidity, the process comprising contacting said soil containing insoluble iron and having any level of humidity with a microorganism of the invention. In a particular embodiment, said process comprises:
injecting, one or more times, a culture of a microorganism of the invention into said soil containing insoluble iron and having any level of humidity; or alternatively
adding to said soil containing insoluble iron and having any level of humidity an active solid support of the invention; or alternatively
adding to said soil containing insoluble iron and having any level of humidity a supplemented fertilizer of the invention; or alternatively
sowing said soil containing insoluble iron and having any level of humidity with a supplemented seed of the invention.

In a specific embodiment, said process for the microbiological solubilization, under aerobic conditions, of insoluble iron present in a soil containing insoluble iron and having any level of humidity is carried out by means of injecting microorganisms of the invention into the soil to be treated, for example, by means of a process comprising:
a) the primary injection into said soil of a culture containing a microorganism of the invention; and
b) successive injections of cultures containing microorganisms of the invention until the complete or partial solubilization of the insoluble iron present in the soil to be treated.

Virtually any soil containing insoluble iron (e.g., insoluble iron of the iron oxide type or any another form of iron) can be treated according to this process for removing all or part of the insoluble iron for completely or partially reducing the insoluble iron content present in the soil to be treated; nevertheless in a particular embodiment, said soil containing insoluble iron to be treated is a soil, such as an agricultural topsoil, a topsoil treated with fertilizers, etc. If necessary, the soil to be treated is supplemented with a carbon source and/or with a nitrogen source and/or with essential nutrients to facilitate survival of the microorganisms of the invention.

As it has been previously mentioned, due to the particulars of the microorganism of the invention which is extremely tolerant towards dessication and remains viable and functional at extreme low humidity conditions, as in soils with only 2% field capacity, the level of humidity of the soil can vary within a broad range (high, normal, low or extreme low humidity conditions); thus, soils with 50% field capacity or less, such as soils with 20% field capacity or less, e.g., soils with 10% field capacity or less, even soils with 2% field capacity can be used for carrying out the above defined process, as well as soils with more than 50% field capacity.

The microorganisms of the invention are injected for the purpose of reaching high cell density in the soil to be treated, for example equal to or greater than 10³ microorganisms of the invention per gram of soil to be treated, typically equal to or greater than 10⁴ microorganisms of the invention per gram of soil to be treated, preferably equal to or greater than 10⁵ microorganisms of the invention per gram of soil to be treated, for the purpose of facilitating solubilization of the insoluble iron.

In a specific embodiment, said process for the microbiological solubilization, under aerobic conditions, of insoluble iron present in a soil containing insoluble iron and having any level of humidity is carried out by means of adding to said soil to be treated an active solid support of the invention comprising a microorganism of the invention. The features of said active solid support of the invention have been previously described. The amount of active solid support of the invention which is added to the soil to be treated (soil containing insoluble iron and having any level of humidity) may vary within a wide range; nevertheless in a particular embodiment, the amount of said active solid support of the invention which is added to the soil to be treated is equal to or greater than 0.01 kg of active solid support of the invention per hectare (Ha) of soil to be treated, typically equal to or greater than 0.1 kg/Ha, preferably equal to or greater than 0.5 kg/Ha of soil to be treated, even more preferably equal to or greater than 1 kg/Ha of soil to be treated, for the purpose of facilitating solubilization of the insoluble iron.

In another specific embodiment, said process for the microbiological solubilization, under aerobic conditions, of insoluble iron present in a soil containing insoluble iron and having any level of humidity is carried out by means of adding to said soil to be treated a supplemented fertilizer of the invention comprising a microorganism of the invention. The features of said supplemented fertilizer of the invention have been previously described. The amount of supplemented fertilizer of the invention which is added to the soil to be treated (soil containing insoluble iron and having any level of humidity) may vary within a wide range, depending on, among other factors, the amount of insoluble iron present in the soil to be treated and on the amount of microorganisms of the invention present in the supplemented fertilizer of the invention.

In another specific embodiment, said process for the microbiological solubilization, under aerobic conditions, of insoluble iron present in a soil containing insoluble iron and having any level of humidity is carried out by means of sowing said soil to be treated with a seed of the invention comprising a microorganism of the invention. The features of said seed of the invention have been previously described. The amount of supplemented seeds of the invention which are sowed in the soil to be treated (soil containing insoluble iron and having any level of humidity) may vary within a wide range, depending on the density required by the farmer.

The solubilization of iron, for example, by means of chelation by siderophores, prevents the iron from being available for pathogenic microorganisms, particularly plant pathogens, thus exerting biocontrol on agriculturally unwanted microbial populations. This advantage can be obtained by means of the use of the microorganism of the invention.

In another aspect, the invention relates to a process for the microbiological solubilization, under aerobic conditions, of an insoluble phosphate and iron in a solid substrate containing insoluble phosphate and insoluble iron, comprising contacting said solid substrate to be treated with a microorganism of the invention. In a particular embodiment, said microorganism of the invention forms part of an active solid support of the invention. Therefore, in a particular embodiment said process comprises contacting said solid substrate containing insoluble phosphate and insoluble iron to be treated with a culture of a microorganism of the invention; or alternatively with an active solid support of the invention.

In a specific embodiment, said process comprises:
a) inoculating a mixture comprising said solid substrate containing insoluble phosphate and insoluble iron to be treated and water with a culture comprising a microorganism of the invention; and, if desired,
b) removing said microorganism.

Virtually any solid substrate containing insoluble phosphate and insoluble iron can be treated according to this process for completely or partially reducing the insoluble phosphate and insoluble iron content; nevertheless in a particular embodiment, said solid substrate containing insoluble phosphate and insoluble iron to be treated is rock phosphate, such as rock phosphate used to produce phosphate fertilizers with a variable insoluble phosphate and, occasionally, insoluble iron content.

To carry out this process, the solid substrate containing insoluble phosphate and insoluble iron to be treated is mixed with water, and the resulting mixture is contacted with a culture of a microorganism of the invention, or with an active solid support of the invention, at a bacterial population density which may vary within a wide range; nevertheless in a particular embodiment, at least 10³ microorganisms of the invention per ml of water are inoculated. After inoculation, the microorganisms of the invention are left to act and after 1 to 24 hours, if desired, said microorganisms are removed by conventional methods, for example by means of decantation, precipitation with electrolytes, centrifugation, etc. If desired, the removed microorganisms of the invention can be reused in a new process for the microbiological solubilization, under aerobic conditions, of an insoluble phosphate and/or insoluble iron in a solid substrate containing insoluble phosphate and/or insoluble iron.

This process can be carried out in a reactor, pond or the like, in which the solid substrate containing insoluble phosphate and insoluble iron to be treated is introduced, duly equipped with means for feeding and draining water, solid substrate, inoculation of microorganisms of the invention and recovery of the same. If necessary, the mixture (solid substrate, water and microorganisms of the invention optionally forming part of an active solid support of the invention) is supplemented with a carbon source and/or with a nitrogen source and/or essential nutrients, for the purpose of facilitating survival of the microorganisms of the invention. By way of illustration, suitable amounts of micronutrient solution along with suitable amounts of magnesium, cobalt and molybdenum, typically in the micromolar order, can be added to optimize the process; nevertheless, in any case the choice and amount of nutrients and micronutrients to be added will depend on the composition of the solid substrate to be treated and on the microbiological demand.

This process can be used to solubilize all or part of the insoluble phosphate and of the insoluble iron present in the solid substrate to be treated for the purpose of reducing the insoluble phosphate and insoluble iron content present in said solid substrate to be treated.

In another aspect, the invention relates to a process for the microbiological solubilization, under aerobic conditions, of insoluble phosphate and insoluble iron present in a soil containing insoluble phosphate and insoluble iron, wherein said soil has any level of humidity, the process comprising contacting said soil containing insoluble phosphate and insoluble iron to be treated with a microorganism of the invention. Briefly, in a particular embodiment said process comprises:
injecting, one or more times, a culture of a microorganism of the invention into said soil containing insoluble phosphate and insoluble iron and having any level of humidity; or alternatively
adding to said soil containing insoluble phosphate and insoluble iron and having any level of humidity an active solid support of the invention; or alternatively
adding to said soil containing insoluble phosphate and insoluble iron and having any level of humidity a supplemented fertilizer of the invention; or alternatively
sowing said soil containing insoluble phosphate and insoluble iron and having any level of humidity with a supplemented seed of the invention.

In a specific embodiment, said process for the microbiological solubilization, under aerobic conditions, of insoluble phosphate and insoluble iron present in a soil containing insoluble phosphate and insoluble iron and having any level of humidity is carried out by means of injecting microorganisms of the invention into the soil to be treated, for example, by means of a process comprising:
a) the primary injection into said soil of a culture containing a microorganism of the invention; and
b) successive injections of cultures containing microorganisms of the invention until the complete or partial solubilization of the insoluble phosphate and of the insoluble iron present in the soil to be treated.

Virtually any soil containing an insoluble phosphate (e.g., dibasic calcium phosphate, tribasic calcium phosphate, etc., and mixtures thereof) and insoluble iron (e.g., insoluble iron of the iron oxide type or any other form of iron) can be treated according to this process for completely or partially reducing the insoluble phosphate content and in insoluble iron present in said soil to be treated; nevertheless in a particular embodiment, said soil is a agricultural topsoil, a topsoil treated with fertilizers, etc. If necessary, the soil to be treated is supplemented with a carbon source and/or with a nitrogen source and/or with essential nutrients to facilitate survival of the microorganisms of the invention.

As it has been previously mentioned, due to the particulars of the microorganism of the invention which is extremely tolerant towards dessication and remains viable and functional at extreme low humidity conditions, as in soils with only 2% field capacity, the level of humidity of the soil can vary within a broad range (high, normal, low or extreme low humidity conditions); thus, soils with 50% field capacity or less, such as soils with 20% field capacity or less, e.g., soils with 10% field capacity or less, even soils with 2% field capacity can be used for carrying out the above defined process, as well as soils with more than 50% field capacity.

The microorganisms of the invention are injected for the purpose of reaching high cell density in the soil to be treated, for example, equal to or greater than 10³ microorganisms of the invention per gram of soil to be treated, typically equal to or greater than 10⁴ microorganisms of the invention per gram of soil to be treated, preferably equal to or greater than 10⁵ microorganisms of the invention per gram of soil to be treated, for the purpose of facilitating solubilization of the insoluble phosphate and of the insoluble iron.

In a specific embodiment, said process for the microbiological solubilization, under aerobic conditions, of insoluble phosphate and insoluble iron present in a soil containing an insoluble phosphate and insoluble iron and having any level of humidity is carried out by means of adding to said soil to be treated an active solid support of the invention comprising a microorganism of the invention. The features of said active solid support of the invention have been previously described. The amount of active solid support of the invention which is added to the soil to be treated may vary within a wide range; nevertheless in a particular embodiment, the amount of said active solid support of the invention which is added to the soil to be treated is equal to or greater than 0.01 kg of active solid support of the invention per hectare (Ha) of soil to be treated, typically equal to or greater than 0.1 kg/Ha, preferably equal to or greater than 0.5 kg/Ha of soil to be treated, even more preferably equal to or greater than 1 kg/Ha of soil to be treated, for the purpose of facilitating solubilization of the insoluble phosphate and of the insoluble iron.

In another specific embodiment, said process for the microbiological solubilization, under aerobic conditions, of insoluble phosphate and insoluble iron present in a soil containing an insoluble phosphate and insoluble iron and having any level of humidity is carried out by means of adding to said soil to be treated a supplemented fertilizer of the invention comprising a microorganism of the invention. The features of said supplemented fertilizer of the invention have been previously described. The amount of supplemented fertilizer of the invention which is added to the soil to be treated may vary within a wide range, depending on, among other factors, the amount of insoluble phosphate present in the soil to be treated and on the amount of microorganisms of the invention present in the supplemented fertilizer of the invention.

In another specific embodiment, said process for the microbiological solubilization, under aerobic conditions, of insoluble phosphate and insoluble iron present in a soil containing an insoluble phosphate and insoluble iron and having any level of humidity is carried out by means of sowing said soil to be treated with a seed of the invention comprising a microorganism of the invention. The features of said seed of the invention have been previously described. The amount of supplemented seeds of the invention which are sowed in the soil to be treated may vary within a wide range, depending on the density required by the farmer.

The previously described processes for the microbiological solubilization of insoluble phosphate and insoluble iron provide, among others, the previously mentioned advantages of high specificity, working in a wide range of phosphate concentrations, typically between 0.01% and 95% (w/w) phosphate, and high versatility; likewise, the solubilization of iron, for example by means of chelation by siderophores, prevents the iron from being available for pathogenic microorganisms, particularly plant pathogens, thus exerting biocontrol on agriculturally unwanted microbial populations.

In another aspect, the invention relates to a process for stimulating plant growth, comprising the application of an effective amount of a microorganism of the invention to the soil surrounding said plant.

Virtually any plant can be treated according to this process for stimulating its growth, particularly its growth in early stages; by way of a non-limiting illustrative example, said plant can be a plant of agricultural interest, including plants of interest in human or animal consumption, plants of a decorative interest, plants of an energy interest, etc. (oats, barley, corn, wheat, grass, sorghum, rose bushes, geraniums, daisies, etc.).

The microorganism of the invention can be applied in different ways. In a particular embodiment, said microorganism of the invention is applied directly to the soil, whereas in another particular embodiment, the microorganism of the invention is applied adhered to a solid support in the form of an active solid support of the invention. Likewise in another particular embodiment, the microorganism of the invention is applied by coating all or part of the seeds of the plants the growth of which is to be stimulated, in the form of a supplemented seed of the invention. Alternatively, in another particular embodiment, the microorganism of the invention is applied along with a fertilizer in the soil surrounding the plants the growth of which is to be stimulated, in the form of a supplemented fertilizer of the invention.

The features of said active solid support, supplemented seed and supplemented fertilizer provided by this invention have been previously described.

The effective amount of microorganism of the invention to be applied to the soil surrounding the plant the growth of which is to be stimulated may vary within a wide range; nevertheless in a particular embodiment, the amount of microorganism of the invention to be applied to the soil surrounding the plant the growth of which is to be stimulated is comprised between 10³ and 10⁸ microorganisms per gram of soil adjacent to the root system. The presence of microorganisms promotes the simultaneous seed germination, gives a homogenous appearance to plant growth and coordinates the fruit flowering and maturation period.

The following examples illustrate the invention and should not be interpreted so as to limit the scope of the invention.

### EXAMPLE 1

### Isolation of the bacteria of the invention

A sample of 10 g of a soil from a garden in Pinos Genil (Granada) was suspended in a modified A medium, at a 1:10 ratio (w/v). Said modified A medium is a minimal medium without a phosphorus source the specific composition per liter of which is: NH₄Cl, 267 mg; MgSO₄.7H₂O, 410 mg; KCl, 300 mg; NaCl, 200 mg; and 1 ml of an aqueous solution of iron citrate (6 g/l). Tribasic calcium phosphate was subsequently added to said medium in an amount sufficient to reach a concentration of 10% (w/v).

The resulting suspension was incubated at a temperature comprised between 25°C and 30°C, with stirring, for a week under aerobic conditions. Suitable dilutions of said suspension were subsequently spread on plates of selective solid medium formed by modified A medium, 1.5% (w/v) agar and 1% (w/v) benzoate.

After incubating between 1 and 7 days, the isolated colonies were purified and their phenotype was verified by culturing them in the aforementioned medium. Several colonies of bacteria which were classified as *Pseudomonas putida* which had the insoluble phosphate (tribasic calcium phosphate) solubilizing capacity were identified following microbiology-type analysis (gram staining, catalase assay, oxidase assay, nutrient use test, etc.).

Then the capacity of said bacteria of using iron in trace amounts (as an Fe source for said bacteria), under aerobic conditions, was analyzed. To that end, the previously isolated bacteria classified as P. *putida* were individually inoculated in a M9-type medium (Abril, M.A., Michán, C., Timmis, K.N. and Ramos, J.L. Regulator and enzyme specificities of TOL plasmid-encoded upper pathway for degradation of aromatic hydrocarbons. J. Bacteriol. (1989), 171:6782-6790) without the addition of any iron. The cultures were incubated with stirring (between 50 and 200 rpm) in a Kühner-type orbital incubator. Viable microorganisms were counted after 24 hours (firstly) and subsequently every 24 hours over the time of the assay, observing that the population density doubled approximately every 6 hours until cell densities in the order of 10⁸ colony forming units per milliliter (cfu/ml) were reached, at which time the rate of growth dropped, although the cultures reached up to 10⁹ cfu/ml after 24 hours of incubation. The supernatants of the cultures acquired a greenish-yellow color typical of the excretion of siderophores into the culture medium.

A culture of bacteria from the colony identified as *P*. *putida* BIRD-1 was deposited on June 3, 2008 in the Spanish Type Culture Collection (CECT), Burjasot (Valencia, Spain), with access number CECT 7415.

### EXAMPLE 2

### Use of tribasic calcium phosphate as a phosphorus source for the bacteria of the invention under aerobic conditions

A culture of *P*. *putida* BIRD-1 (CECT 7415) [Example 1] with 10³ microorganisms was inoculated in a modified A medium [specific composition per liter: NH₄Cl, 267 mg; MgSO₄.7H₂O, 410 mg; KCl, 300 mg; NaCl, 200 mg; and 1 ml of an aqueous solution of iron citrate (6 g/l)] with 1% (w/w) tribasic calcium phosphate and was incubated with stirring (between 50 and 200 rpm) in a Kühner-type orbital incubator. Viable microorganisms were counted over time (every 24 h), observing that the bacterial population density doubled approximately every 2 hours until cell densities in the order of 10⁸ cfu/ml were reached, at which time the rate of growth dropped although the cultures reached up to 10⁹ cfu/ml after 24 hours of incubation.

### EXAMPLE 3

### Use of insoluble phosphates as a phosphorus source for the bacteria of the invention under aerobic conditions

The process described in Example 2 was repeated but using ground rock phosphate containing from 15 to 30% P₂O₅ and the grain size of which was less than 3 mm; 0.1 to 5% (w/v) of rock phosphate was added, observing that the rate of duplication of the bacterial population was slower and in the order of 4 hours; nevertheless, the cell cultures reached between 10¹⁰ and 10¹¹ cfu/ml.

### EXAMPLE 4

### Use of iron in trace amounts in rock phosphate as an iron source for the bacteria of the invention under aerobic conditions

A culture of *P. putida* BIRD-1 (CECT 7415) [Example 1] with 10³ microorganisms was inoculated in a M9-type medium (Abril, M.A., Michán, C., Timmis, K.N. and Ramos, J.L. Regulator and enzyme specificities of TOL plasmid-encoded upper pathway for degradation of aromatic hydrocarbons. J. Bacteriol. (1989), 171:6782-6790) without the addition of any iron to which 1% (w/w) ground rock phosphate containing from 15 to 30% of P₂O₅ was added and the grain size of which was less than 3 mm, and it was incubated with stirring (between 50 and 200 rpm) in a Kühner-type orbital incubator. The viable microorganisms were counted over time every 24 hours, observing that the population density doubled approximately every 6 hours until cell densities in the order of 10⁸ colony forming units per milliliter (cfu/ml) were reached, at which time the rate of growth dropped although the cultures reached up to 10⁹ cfu/ml after 24 hours of incubation. The supernatant of the culture acquired a greenish-yellow color typical of the excretion of siderophores into the culture medium.

### EXAMPLE 5

### Pseudomonas putida BIRD-1 exhibits unique features

The *rpoD, gyrB, trpE, gltA y edd* were partially amplified and compared with sequences deposited in databanks. The sequences of the corresponding genes exhibited high similarity with genes from bacteria of the genus *Pseudomonas,* but with significant differences, indicating the existence of genetic variability. The results are shown in Tables 1 and 2.

**Table 1**

| **Gene** | **Gene size (bp)** | **Amplified fragment (bp)** | **Non-identical bp in the amplified fragment with the closest organism** | **% identity with BIRD-1** |
|---|---|---|---|---|
| gltA (Citrate Synthase) | 1290 | 1190 | 7 | *Pseudomonas putida* KT2440 **(99%)** |
| | | | | *Pseudomonas putida* F1 **(98%)** |
| | | | | *Pseudomonas putida* GB-1 **(97%)** |
| | | | | *Pseudomonas putida* W619 **(94%)** |
| trpE (Anthranilate Synthase, component I) | 1482 | 1382 | 8 | *Pseudomonas putida* F1 **(99%)** |
| | | | | *Pseudomonas putida* KT2440 **(98%)** |
| | | | | *Pseudomonas putida* GB-1 **(92%)** |
| | | | | *Pseudomonas putida* W619 **(82%)** |
| | | | | |

**Table 2**

| **Gene** | **% identity with respect to the closest sequence and its source** |
|---|---|
| *RpoD* | 99% *P. putida* KT2440 |
| | 99% *P. putida* F1 |
| | *97% P. putida* |
| *Edd* | 98% *P. putida* KT2440 |
| *gltA* | 99% *P. putida* KT2440 |
| *trpE* | 97% *P. putida* KT2440 |
| *gyrB* | 98% *P. putida* KT2440 |

### EXAMPLE 6

### Pseudomonas putida BIND-1 tolerates extremely low humidity conditions

To assess tolerance towards desiccation, 10⁷ cfu of BIRD-1 were added to soils whose humidity had been prefixed at 50, 20, 10 and 2% (field capacity). The number of viable cells of the strain was maintained for 15 days at 10⁷ to 10⁸ cfu/g of soil, regardless of the humidity rate. In parallel controls with strains such as *P. putida* KT2440 (Abril et al. cited *supra),* viability only occurred at relative humidity levels above 10%.

### EXAMPLE 7

### Barley seed germination stimulation

250 barley seeds were sowed in Petri dishes with 40 g of agricultural-type soil of pH 7.5 which had previously been mixed with *P. putida* BIRD-1 bacteria (CECT 7415) [Example 1] to reach 10⁶ cfu/g soil.

250 barley seeds were sowed in Petri dishes with 40 g of the same agricultural soil of pH 7.5 without *P*. *putida* BIRD-1 bacteria (CECT 7415) [Example 1] as a control.

Seed germination ended after 4 days of incubation at 20°C in the dark. It was observed that the percentage of barley seed germination in the agricultural soil without bacteria was 66.6%, whereas it reached at least 98.6% in the soil treated with said *P. putida* BIRD-1 bacteria (CECT 7415) [Example 1].

### EXAMPLE 8

### Grass seed germination stimulation

The assay described in Example 7 was repeated but using grass seeds instead of barley seeds, observing that the percentage of grass seed germination in the soil without bacteria was 92%, whereas it reached 100% in the soil treated with said *P. putida* BIRD-1 bacteria (CECT 7415) [Example 1]. It was additionally observed that the root system of all the plants is in the order of 1.3 to 2 times denser than that of the seeds which have not been inoculated.

### EXAMPLE 9

### Corn seed germination stimulation

The assay described in Example 7 was repeated but using corn seeds instead of barley seeds, observing that the percentage of corn seed germination in the soil without bacteria was 93%, whereas it reached the 100% in the soil treated with said *P. putida* BIRD-1 bacteria (CECT 7415) [Example 1].

### EXAMPLE 10

### Early corn plant growth stimulation

To determine the plant growth-stimulating potential of the *P. putida* BIRD-1 bacteria (CECT 7415) [Example 1] on a solid support (sepiolite) several greenhouse assays were performed in which an agricultural soil was used which was mixed with silica sand and with sepiolite on which said bacteria were adhered to reach a final density of at least 10⁵ microorganisms per gram of mixture, in 2:1:1; 2:1:0.1 and 2:1:0.01 [agricultural soil:silica sand:sepiolite containing *P*. *putida* BIRD-1 (CECT 7415)] mixtures. Corn seeds were planted on said well homogenized mixtures, and the percentage of germination and the stem length over time was determined a week after sowing.

As a control, corn seeds were sowed in agricultural soil mixed with silica sand and with solid supports (sepiolite) without bacteria in 2:1:1; 2:1:0.1 and 2:1:0.01 [agricultural soil:silica sand:solid support] mixtures. Corn seeds were planted on said well homogenized mixtures, and the percentage of germination and the stem length over time was determined a week after sowing.

It was observed that the stems of the corn plants in the cultures with bacteria were between 7% and 10% longer than the stems of the corn plants sowed in soils without bacteria, although after 20 days of growth the stem lengths of the corn plants were identical.

### EXAMPLE 11

### Early ryegrass growth stimulation

The assay of Example 10 was repeated but sowing ryegrass instead of corn, observing that the stems were of the same length both in the presence and absence of bacteria; nevertheless, in the presence of *P*. *putida* BIRD-1 bacteria (CECT 7415), the length of the root reached 3 cm whereas in the absence of bacteria it was only 2.3 cm in length.

### EXAMPLE 12

### Solubilization of rock phosphate phosphorus

10 g of rock phosphate with 27% P₂O₅ were introduced in a vessel containing 100 ml of water and was supplemented with the micronutrient solution described by Abril et al. (Abril, M.A., Michán, C., Timmis, K.N. and Ramos, J.L. Regulator and enzyme specificities of TOL plasmid-encoded upper pathway for degradation of aromatic hydrocarbons. J. Bacteriol. (1989), 171:6782-6790) and at least 10³ *P. putida* BIRD-1 bacteria (CECT 7415) [Example 1] and 1% (w/v) sucrose. The system was aerated by means of air pumping. The phosphorus used was determined every 24 hours and when 1 to 5% of the total phosphorus had been solubilized after 24 hours, increasing up to about 20% in an additional 24 to 96 hours, the suspension was removed and the processes was repeated.

### EXAMPLE 13

### Solubilization of phosphate from phosphoric rock at low humidity (2%)

To establish phosphate solubilization in phosphoric rock, 10 g of dry weight phosphoric rock were mixed with 27% P₂O₅ with 10 g of oven-dried soil at 270°C. The mixture was supplemented with 10⁷ cfu/g of suspended mixture in 0.5 ml of water. After mixing well and reaching homogenisation, the flask was sealed with a Teflon tampon and cultured at room temperature (18 to 22°C) for 10 days. A control assay was set up in parallel but without microorganisms. Soluble phosphate was determined using a fraction of the sample, revealing that almost 1% of total phosphate was soluble, whereas the levels in the control sample were lower than 0.1%.

### EXAMPLE 14

### Solubilization of phosphate in soils with average humidity levels

As in Example 13 except that the water content was 10%. The level of solubilized phosphate reached 3% of the total in 10 days.

### EXAMPLE 15

### Solid fertilizer complement

Formulations of NPK-type fertilizers (12:12:12 and 4:8:12) and fulvic acid-based liquid solutions with 10⁶ to 10⁸ cfu/ml of *P. putida* BIRD-1 bacteria (CECT 7415) immobilized in sepiolite at a ratio of 1:100 were mixed together. The supplemented fertilizer with said bacteria was used to stimulate corn plant growth.

### EXAMPLE 16

### Solid fertilizer complement by adhesion

The same process was used as in Example 11 but using 0.1% acacia gum to facilitate adhesion of *P. putida* BIRD-1 bacteria (CECT 7415) to the solid fertilizers.

## Claims

1. A microorganism of the *Pseudomonas putida* species deposited in the Spanish Type Culture Collection (CECT) with access number. CECT 7415, having phosphate and iron solubilizing capacity in conditions wherein the amount of water is 20% or lower, or a mutant of said microorganism maintaining phosphate and iron solubilizing capacity in conditions wherein the amount of water is 20% or lower.

2. The microorganism according to claim 1, further producing a plant growth-stimulating compound in conditions wherein the amount of water is 20% or lower, wherein said plant growth-stimulating compound is a plant hormone.

3. A biologically pure culture of a microorganism according to any of claims 1 or 2.

4. An active solid support comprising a solid support and a microorganism according to any of claims 1 or 2.

5. A supplemented fertilizer comprising a fertilizer and a microorganism according to any of claims 1 or 2.

6. A supplemented seed comprising a seed and a microorganism according to any of claims 1 or 2, optionally adhered to a solid support.

7. A process for the microbiological solubilization, under aerobic conditions, of an insoluble phosphate in a solid substrate containing an insoluble phosphate, comprising contacting said solid substrate with a microorganism according to any of claims 1 or 2, or with an active solid support claim 4.

8. The process according to claim 7, wherein said solid substrate is rock phosphate.

9. A process for the microbiological solubilization, under aerobic conditions, of insoluble phosphate present in a soil containing an insoluble phosphate, wherein said soil has any level of humidity, the process comprising
injecting one or more times a culture of a microorganism according to any of claims 1 or 2 into said soil; or alternatively
adding to said soil an active solid support according to claim 4; or alternatively
adding to said soil a supplemented fertilizer according to claim 5; or alternatively
seeding said soil with a supplemented seed according to claim 6.

10. A process for the microbiological solubilization, under aerobic conditions, of insoluble iron present in a solid substrate containing insoluble iron, comprising contacting said solid substrate with a microorganism according to any of claims 1 or 2, or with an active solid support according to claim 4.

11. A process for the microbiological solubilization, under aerobic conditions, of insoluble iron present in a soil containing insoluble iron, wherein said soil has any level of humidity, the process comprising
injecting one or more times a culture of a microorganism according to any of claims 1 or 2 into said soil; or alternatively
adding to said soil an active solid support according to claim 4; or alternatively
adding to said soil a supplemented fertilizer according to claim 5; or alternatively
seeding said soil with a supplemented seed according to claim 6.

12. A process for the microbiological solubilization, under aerobic conditions, of an insoluble phosphate and of insoluble iron in a solid substrate containing an insoluble phosphate and insoluble iron, comprising contacting said solid substrate with a microorganism according to any of claims 1 or 2, or with an active solid support claim 4.

13. A process for the microbiological solubilization, under aerobic conditions, of insoluble phosphate and insoluble iron present in a soil containing an insoluble phosphate and insoluble iron, wherein said soil has any level of humidity, the process comprising
injecting one or more times a culture of a microorganism according to any of claims 1 or 2 into said soil; or alternatively
adding to said soil an active solid support according to claim 4; or alternatively
adding to said soil a supplemented fertilizer according to claim 5; or alternatively
seeding said soil with a supplemented seed according to claim 6.

14. A process for promoting plant growth, comprising applying an effective amount of a microorganism according to any of claims 1 or 2, or of an active solid support according to claim 4, or of a supplemented fertilizer according to claim 5, to the soil surrounding said plant, or alternatively
seeding a seed of said plant supplemented with a microorganism according to any of claims 1 or 2, or with an active solid support according to claim 4.

15. A process for isolating a microorganism having phosphate and iron solubilizing capacity in conditions wherein the amount of water is 20% or lower, comprising:
a) suspending a sample of a soil suspected of containing said microorganism having phosphate and iron solubilizing capacity in a culture medium lacking a phosphorus source;
b) adding to the suspension obtained in step a) a phosphorus source formed by one or more insoluble phosphates, a carbon source and a nitrogen source;
c) incubating the suspension resulting from step b) under conditions which allow the growth of said microorganisms which solubilize the insoluble phosphate;
d) seeding dilutions of the suspension resulting from step c) in plates of selective solid medium containing up to 1% (w/w) of an insoluble phosphate as the sole phosphorus source;
e) incubating the spread plates of step d) during a time period comprised between 1 and 7 days at a temperature comprised between 15°C and 42°C;
f) isolating and purifying the colonies of the microorganisms which, obtained after the incubation of step e), are capable of using insoluble phosphate under aerobic conditions;
g) optionally selecting and, if desired, characterizing the strains of the colonies of microorganisms isolated and purified in step f);
h) spread on plates colonies of the microorganisms which, isolated and purified in step f) or optionally selected in step g), are capable of using insoluble phosphate under aerobic conditions, in an iron-deficient culture medium containing a carbon source and a nitrogen source;
i) incubating the spread plates of step h) at a temperature comprised between 15°C and 42°C during a time period comprised between 1 and 7 days, under conditions which allow the growth of microorganisms which solubilize iron;
j) seeding dilutions of the suspension resulting from step i) in plates of iron-deficient solid culture medium;
k) incubating the spread plates of step j) during a time period comprised between 1 and 7 days at a temperature comprised between 15°C and 42°C;
l) isolating and purifying the colonies of microorganisms which, obtained after the incubation of step k), are capable of obtaining iron from the trace concentrations present in the culture media and under aerobic conditions;
m) selecting and optionally characterizing the strains of the colonies of the microorganisms isolated and purified in step l); and
n) subjecting the strains of the colonies of the microorganisms selected and optionally **characterized in** step m) to an assay to assess tolerance towards desiccation and selecting those strains having phosphate and iron solubilizing capacity in conditions wherein the amount of water is 20% or lower.

## Patentansprüche

1. Zu der Spezies *Pseudomonas putida* gehörender Mikroorganismus, hinterlegt bei der Spanischen Kultursammlung (CECT) mit der Referenznummer CECT 7415, der phosphatund eisensolubilisierende Fähigkeiten unter Bedingungen aufweist, unter denen der Wassergehalt 20 % oder weniger ist, oder eine Mutante dieses Mikroorganismus mit erhalten gebliebenen phosphat- und eisensolubilisierenden Fähigkeiten unter Bedingungen, unter denen der Wassergehalt 20 % oder weniger ist.

2. Mikroorganismus gemäß Anspruch 1, der ferner eine Pflanzenwachstum stimulierende Verbindung unter Bedingungen herstellt, unter denen der Wassergehalt 20 % oder weniger ist, wobei die Pflanzenwachstum stimulierende Verbindung ein Pflanzenhormon ist.

3. Biologisch reine Kultur eines Mikroorganismus gemäß einem der Ansprüche 1 oder 2.

4. Aktiver, fester Träger, der einen festen Träger und einen Mikroorganismus gemäß einem der Ansprüche 1 oder 2 umfasst.

5. Ergänzter Dünger, der einen Dünger und einen Mikroorganismus gemäß einem der Ansprüche 1 oder 2 umfasst.

6. Ergänzter Samen, der einen Samen und einen Mikroorganismus gemäß einem der Ansprüche 1 oder 2, optional gebunden an einen festen Träger, umfasst.

7. Verfahren zur mikrobiologischen Solubilisation unter aeroben Bedingungen von unlöslichem Phosphat in einem festen Substrat, das unlösliches Phosphat enthält, bei dem das feste Substrat mit einem Mikroorganismus gemäß einem der Ansprüche 1 oder 2, oder mit einem aktiven, festen Träger gemäß Anspruch 4 kontaktiert wird.

8. Verfahren gemäß Anspruch 7, wobei das feste Substrat Phosphatgestein ist.

9. Verfahren zur mikrobiologischen Solubilisation unter aeroben Bedingungen von unlöslichem Phosphat, das in einem Boden vorhanden ist, der unlösliches Phosphat enthält, wobei der Boden ein beliebiges Feuchtigkeitsniveau aufweist, wobei in dem Verfahren einmalig oder mehrfach eine Kultur eines Mikroorganismus gemäß einem der Ansprüche 1 oder 2 in den Boden injiziert wird; oder alternativ dem Boden ein aktiver, fester Trägers gemäß Anspruch 4 zugegeben wird; oder alternativ dem Boden ein ergänzter Dünger gemäß Anspruch 5 zugegeben wird; oder alternativ in den Boden ein ergänzter Samen gemäß Anspruch 6 gesät wird.

10. Verfahren zur mikrobiologischen Solubilisation unter aeroben Bedingungen von unlöslichem Eisen, das in einem festen Substrat vorhanden ist, das unlösliches Eisen enthält, bei dem das feste Substrat mit einem Mikroorganismus gemäß einem der Ansprüche 1 oder 2 oder mit einem aktiven festen Träger gemäß Anspruch 4 kontaktiert wird.

11. Verfahren zur mikrobiologischen Solubilisation unter aeroben Bedingungen von unlöslichem Eisen, das in einem Boden vorhanden ist, der unlösliches Eisen enthält, wobei der Boden ein beliebiges Feuchtigkeitsniveau aufweist, wobei in dem Verfahren einmalig oder mehrfach eine Kultur eines Mikroorganismus gemäß einem der Ansprüche 1 oder 2 in den Boden injiziert wird; oder alternativ dem Boden ein aktiver, fester Träger gemäß Anspruch 4 zugegeben wird; oder alternativ dem Boden ein ergänzter Dünger gemäß Anspruch 5 zugegeben wird; oder alternativ in den Boden ein ergänzter Samen gemäß Anspruch 6 gesät wird.

12. Verfahren zur mikrobiologischen Solubilisation unter aeroben Bedingungen von unlöslichem Eisen und unlöslichem Phosphat in einem festen Substrat, das unlösliches Eisen und unlösliches Phosphat enthält, wobei das feste Substrat mit einem Mikroorganismus gemäß einem der Ansprüche 1 oder 2 oder mit einem aktiven, festen Träger gemäß Anspruch 4 kontaktiert wird.

13. Verfahren zur mikrobiologischen Solubilisation unter aeroben Bedingungen von unlöslichem Eisen und unlöslichem Phosphat, die in einem Boden vorhanden sind, der unlösliches Eisen und unlösliches Eisen enthält, wobei der Boden ein beliebiges Feuchtigkeitsniveau aufweist, wobei in dem Verfahren einmalig oder mehrfach eine Kultur eines Mikroorganismus gemäß einem der Ansprüche 1 oder 2 in den Boden injiziert wird; oder alternativ dem Boden ein aktiver, fester Träger gemäß Anspruch 4 zugegeben wird; oder alternativ dem Boden ein ergänzter Dünger gemäß Anspruch 5 zugegeben wird; oder alternativ in den Boden ein ergänzter Samen gemäß Anspruch 6 gesät wird.

14. Verfahren zur Förderung des Pflanzenwachstums, wobei eine wirksame Menge eines Mikroorganismus gemäß einem der Ansprüche 1 oder 2 oder eines aktiven, festen Trägers gemäß Anspruch 4 oder eines ergänzten Düngers gemäß Anspruch 5 dem Boden zugefügt wird, der die Pflanze umgibt, oder alternativ ein Same der Pflanze ausgesät wird, der mit einem Mikroorganismus gemäß einem der Ansprüche 1 oder 2 oder mit einem aktiven festen Träger gemäß Anspruch 4 ergänzt ist.

15. Verfahren zur Isolierung eines Mikroorganismus mit phosphat- und eisensolubilisierenden Fähigkeiten unter Bedingungen, unter denen der Wassergehalt 20 % oder weniger ist, umfassend:
a) Suspendieren einer Probe eines Bodens, von dem vermutet wird, dass er den Mikroorganismus mit eisen- und phosphatsolubilisierenden Fähigkeiten enthält, in einem Kulturmedium, dem eine Phosphorquelle fehlt;
b) Zugabe einer Phosphorquelle, die durch ein oder mehrere unlösliche Phosphate gebildet ist, einer Kohlenstoffquelle und einer Stickstoffquelle zu der in Schritt a) erhaltenen Suspension;
c) Inkubation der aus Schritt b) erhaltenen Suspension unter Bedingungen, die das Wachstum der Mikroorganismen ermöglichen, die das unlösliche Phosphat solubilisieren;
d) Beimpfen von Platten mit selektivem festen Medium, das bis zu 1 % (Gewicht/Gewicht) unlösliches Phosphat als einzige Phosphorquelle enthält, mit Verdünnungen der aus Schritt c) erhaltenen Suspension;
e) Inkubation aus Schritt d) erhaltenen spread-plate-Platten für einen Zeitraum zwischen 1 und 7 Tagen bei einer Temperatur zwischen 15 und 42 °C;
f) Isolierung und Reinigung der Kolonien der Mikroorganismen, die nach dem Inkubationsschritt e) erhalten wurden und fähig sind, unlösliches Phosphat unter aeroben Bedingungen zu nutzen;
g) optionales Selektieren und gewünschtenfalls Charakterisieren der Stämme der in Schritt f) isolierten und gereinigten Kolonien der Mikroorganismen;
h) Ausplattieren der in Schritt f) isolierten und gereinigten oder optional in Schritt g) selektierten Kolonien der Mikroorganismen, die fähig sind, unlösliches Phosphat unter aeroben Bedingungen zu nutzen, in einem eisendefizienten Kulturmedium, das eine Kohlenstoff- und Stickstoffquelle enthält;
i) Inkubation der spread-plate-Platten aus Schritt h) bei einer Temperatur zwischen 15 und 42 °C für einen Zeitraum zwischen 1 und 7 Tagen unter Bedingungen, die das Wachstum der Mikroorganismen ermöglichen, die Eisen solubilisieren;
j) Beimpfen von Platten mit eisendefizientem festem Kulturmedium mit Verdünnungen der in Schritt i) erhaltenen Suspensionen ;
k) Inkubation der spread plate-Platten aus Schritt j) für einen Zeitraum von 1 bis 7 Tagen bei einer Temperatur zwischen 15 und 42 °C;
l) Isolierung und Reinigung der nach dem Inkubationsschritt k) erhaltenen Kolonien von Mikroorganismen, die fähig sind, Eisen aus den Spurenkonzentrationen im Kulturmedium und unter aeroben Bedingungen zu erhalten;
m) Selektieren und optionales Charakterisieren der in Schritt I) isolierten und gereinigten Stämme der Kolonien der Mikroorganismen;
n) Untersuchen der in Schritt m) selektierten und optional charakterisierten Stämme der Kolonien der Mikroorganismen mit einem Assay, um die Austrocknungstoleranz zu beurteilen, und Selektieren jener Stämme, die phosphat- und eisensolubilisierende Fähigkeiten unter Bedingungen aufweisen, unter denen der Wassergehalt 20 % oder weniger ist.

## Revendications

1. Microorganisme de l'espèce *Pseudomonas putida* déposé dans la Collection espagnole de cultures types (CECT), numéro d'accès CECT 7415, présentant une capacité à solubiliser le fer et le phosphate dans des conditions où la quantité d'eau est de 20 % ou inférieure, ou mutant dudit microorganisme maintenant la capacité à solubiliser le fer et le phosphate dans des conditions où la quantité d'eau est de 20 % ou inférieure.

2. Microorganisme selon la revendication 1, produisant en plus un composé stimulant la croissance des plantes dans des conditions où la quantité d'eau est de 20 % ou inférieure, ledit composé stimulant la croissance étant une hormone végétale.

3. Culture biologiquement pure d'un microorganisme selon l'une quelconque des revendications 1 ou 2.

4. Support actif solide comprenant un support solide et un microorganisme selon l'une quelconque des revendications 1 ou 2.

5. Engrais supplémenté comprenant un engrais et un microorganisme selon l'une quelconque des revendications 1 ou 2.

6. Semence supplémentée comprenant une semence et un microorganisme selon l'une quelconque des revendications 1 ou 2, optionnellement adhérée à un support solide.

7. Procédé pour la solubilisation microbiologique, en conditions aérobies, d'un phosphate insoluble dans un substrat solide contenant un phosphate insoluble, comprenant la mise en contact dudit substrat solide avec un microorganisme selon l'une quelconque des revendications 1 ou 2 ou avec un support solide actif selon la revendication 4.

8. Procédé selon la revendication 7, dans lequel ledit substrat est le phosphate de pierre.

9. Procédé pour la solubilisation microbiologique, en conditions aérobies, du phosphate insoluble présent dans un sol contenant un phosphate insoluble, ledit sol ayant un niveau d'humidité quelconque, le procédé comprenant :
l'injection, une ou plusieurs fois, d'une culture d'un microorganisme selon l'une quelconque des revendications 1 ou 2 dans ledit sol ; ou alternativement,
l'ajout audit sol d'un support actif solide selon la revendication 4 ; ou alternativement
l'ajout audit sol d'un engrais supplémenté selon la revendication 5 ; ou alternativement
l'ensemencement dudit sol avec une semence supplémentée selon la revendication 6.

10. Procédé pour la solubilisation microbiologique, en conditions aérobies, du fer insoluble présent dans un substrat solide contenant du fer insoluble, comprenant la mise en contact dudit substrat solide avec un microorganisme selon l'une quelconque des revendications 1 ou 2, ou avec un support actif solide selon la revendication 4.

11. Procédé pour la solubilisation microbiologique, en conditions aérobies, du fer insoluble présent dans un sol contenant du fer insoluble, ledit sol ayant un niveau d'humidité quelconque, ledit procédé comprenant :
l'injection, une ou plusieurs fois, d'une culture d'un microorganisme selon l'une quelconque des revendications 1 ou 2 dans ledit sol ; ou alternativement l'ajout audit sol d'un support actif solide selon la revendication 4 ; ou alternativement
l'ajout audit sol d'un engrais supplémenté selon la revendication 5 ; ou alternativement
l'ensemencement dudit sol avec une semence supplémentée selon la revendication 6.

12. Procédé pour la solubilisation microbiologique, en conditions aérobies, d'un phosphate insoluble et d'un fer insoluble dans un substrat solide comprenant un phosphate insoluble et un fer insoluble, comprenant la mise en contact dudit substrat solide avec un microorganisme selon l'une quelconque des revendicationss 1 ou 2, ou avec un support solide actif selon la revendication 4.

13. Procédé pour la solubilisation microbiologique, en conditions aérobies, du phosphate insoluble et du fer insoluble présents dans un sol contenant du phosphate insoluble et du fer insoluble, ledit sol ayant un niveau quelconque d'humidité, le procédé comprenant
l'injection, une ou plusieurs fois, d'une culture d'un microorganisme selon l'une quelconque des revendications 1 ou 2 dans ledit sol ; ou alternativement l'ajout audit sol d'un support solide actif selon la revendication 4 ; ou alternativement
l'ajout audit sol d'un engrais supplémenté selon la revendication 5 ; ou alternativement
l'ensemencement dudit sol avec une semence supplémentée selon la revendication 6.

14. Procédé pour favoriser la croissance des plantes, comprenant :
l'application d'une quantité efficace d'un microorganisme selon l'une quelconque des revendications 1 ou 2, ou d'un support solide actif selon la revendication 4, ou d'un engrais supplémenté selon la revendication 5 au sol autour de ladite plante ; ou alternativement
l'ensemencement d'une graine de ladite plante supplémentée avec un microorganisme selon l'une quelconque des revendications 1 ou 2, ou avec un support solide actif selon la revendication 4.

15. Procédé pour isoler un microorganisme présentant une capacité à solubiliser le fer et le phosphate dans des conditions où la quantité d'eau est de 20 % ou inférieure, comprenant :
a) la suspension d'un échantillon d'un sol soupçonné de contenir ledit microorganisme présentant une capacité à solubiliser le phosphate et le fer, dans un milieu de culture sans source de phosphore ;
b) l'ajout à la suspension obtenue à l'étape a) d'une source de phosphore formée par un ou plusieurs phosphates insolubles, une source de carbone et une source d'azote ;
c) l'incubation de la suspension résultant de l'étape b) dans des conditions qui permettent la croissance desdits microorganismes qui solubilisent le phosphate insoluble ;
d) l'ensemencement de dilutions de la suspension résultant de l'étape c) dans des plaques d'un milieu solide sélectif contenant jusqu'à 1 % (m/m) d'un phosphate insoluble en tant que seule source de phosphore ;
e) l'incubation des plaques de l'étape d) pendant une période comprise entre 1 et 7 jours à une température comprise entre 15 °C et 42 °C ;
f) l'isolement et la purification des colonies de microorganismes qui, obtenues après l'incubation de l'étape e), sont capables d'utiliser le phosphate insoluble en conditions aérobies ;
g) éventuellement, la sélection et, si on le souhaite, la caractérisation des souches des colonies de microorganismes isolées et purifiées à l'étape f) ;
h) la mise en plaques des colonies de microorganismes qui, isolées et purifiées à l'étape f) ou éventuellement sélectionnées à l'étape g), sont capables d'utiliser le phosphate insoluble en conditions aérobies, dans un milieu de culture déficient en fer mais contenant une source de carbone et une source d'azote ;
i) l'incubation des plaques de l'étape h) à une température comprise entre 15 °C et 42 °C pendant une période de temps comprise entre 1 et 7 jours, dans des conditions qui permettent la croissance de microorganismes qui solubilisent le fer ;
j) l'ensemencement de dilutions de la suspension résultant de l'étape i) dans des plaques d'un milieu de culture solide déficient en fer ;
k) l'incubation des plaques de l'étape j) pendant une période comprise entre 1 et 7 jours à une température comprise entre 15 °C et 42 °C ;
l) l'isolement et la purification des colonies de microorganismes qui, obtenues après l'incubation de l'étape k), sont capables d'obtenir du fer à partir des concentrations traces présentes dans le milieu de culture et en conditions aérobies ;
m) la sélection et éventuellement la caractérisation des souches des colonies des microorganismes isolées et purifiées à l'étape l) ; et
n) la soumission des souches des colonies des microorganismes sélectionnées et éventuellement caractérisées à l'étape m) à une expérience pour tester la tolérance au dessèchement et la sélection des souches présentant une capacité à solubiliser le fer et le phosphate dans des conditions où la quantité d'eau est de 20 % ou inférieure.
